# EUROPEAN PATENT APPLICATION

(11) **EP 2 431 041 A1**
(43) Date of publication of application: **21.03.2012**
(21) Application number: 10774493.0
(22) Date of filing: 11.05.2010
(51) Int. Cl.: A61K 31/56, A61P 9/00, A61P 9/06, A61P 9/10, A61P 3/06

(54) **PHARMACEUTICAL COMPOSITION FOR TREATING CARDIOVASCULAR DISORDER AND USE THEREOF**

(30) Priority: 15.05.2009 CN 200910059307
(71) Applicant: Chengdu Kanghong Pharmaceutical Co., Ltd., Changdu, Sichuan 610036 (CN)
(72) Inventor: HAO, Xiaofeng, Sichuan 610036 (CN); GUO, Lixin, Sichuan 610036 (CN)
(74) Representative: Golding, Louise Ann
(86) International application number: PCT/CN2010/000671
(87) International publication number: WO 2010/130152

(57) **Abstract**

The present invention provides a pharmaceutical composition for treating cardiovascular disorders comprising 3-1 portion echinocystic acid and 1 portion oleanolic acid as the active components. The present invention also provides a gleditsiae extract comprising echinocystic acid and oleanolic acid of 90.0%-99.9% total weight, wherein the weight ratio of echinocystic acid and oleanolic acid is 3:1-1:3. The preparation method and use of the extract are also provided in the present invention. The pharmaceutical composition in the present invention degrades hepatotoxicity by controlling the ratio of echinocystic acid and oleanolic acid in the composition.

## Description

### TECHNICAL FIELD OF THE INVENTION

The invention relates to medical technique field, particularly to a pharmaceutical composition for treating cardiovascular disorders and uses thereof.

### BACKGROUND OF THE INVENTION

*Fructus gleditsiae* (also known as *Gleditsia sinensis* Lam.) distributes widely in China, which possesses both excellent forestry values and considerably broad application prospects in development of clinical Chinese traditional patent medicine. *Fructus gleditsiae* may form two types of fruits in different developmental conditions, i.e., the mature and plump pod known as Chinese honeylocust fruit (Fructus gleditsiae Sinensis), and the retarded and slim one as Chinese honeylocust abnormal fruit (Fructus gleditsiae abnormalis).

Fan Kehua, et al. suggested protection of a gleditsiae extract against myocardial infarction in canines suffering from myocardial ischemia [Fan Kehua, et al. West China Journal of Pharmaceutical Sciences, 2006, 21(4): 339-342]. Ding Yunlu, et al. investigated protection against an acute myocardial ischemia resulting from ligation of the coronary artery in an anesthetized canine and found that gleditsia saponin was capable of improving myocardial ischemia and reducing area of myocardial infarction [Ding Yunlu, et al. Natural Product Research and Development, 2006, 18: 275-278]. Ding Yunlu, et al. further studied effect of the gleditsia saponin on rat myocardial ischemia and found an excellent efficacy of the gleditsia saponin in improving myocardial ischemia in rats and reducing area of myocardial infarction [Ding Yunlu, et al. Chinese Journal of New Drugs and Clinical Remedies, 2006, 26(2):110-113].

At present, the following literatures involve methods of extracting gleditsia saponin. In CN Patent Application No.: 200910020233.7 with the title of A GLEDITSIA SAPONIN EXTRACT, PREPARATION METHODS FOR THE SAME AND USES THEREOF, a gleditsia saponin extract, preparation methods for the same and uses thereof in treatment of lung cancer, hepatocarcinoma, stomach cancer, carcinoma of large intestine, and leukemia were disclosed. The gleditsia saponin extract was prepared by extracting Chinese honeylocust abnormal fruit or Chinese honeylocust fruit as the raw material with water or ethanol and purifying with a macroporous resin. The total content of saponins was above 60%, and the gleditsia saponin mainly comprises various pentacyclic triterpene saponins. In CN Patent Application No. 02109825.5 with the title of A METHOD FOR EXTRACTING TRITERPENIC ACID FROM FRUCTUS GLEDITSIAE, MEDICAL USE OF TRITERPENIC ACID AND CHINESE MEDICINE FORMULATIONS COMPRISING THE

SAME, a method for extracting triterpenic acid from *Fructus gleditsiae,* medical use of the triterpenic acid and Chinese medicine formulations comprising the same were disclosed, in which Fructus gleditsiae pods were subjected to extraction with 50-70% of ethanol, decoloration, filtration, and ethanol recycling, then diluted with deionized water, purified on a macroporous adsorptive resin column via elution, concentration, and dried to yield total triterpenoid saponins. The extract of the invention can be used in preparation of a drug for treating and preventing diseases, such as hyperlipemia, fatty liver, atheroma, etc.

HAO Yu-fang found the gleditsia saponin has relatively high toxicity, which limits the use thereof in clinical application [HAO Yu-fang, Preparation of Gleditsia Sapogein and Study on its Anti-Myocardial Ischemia Efficacy, Master's Degree thesis, Changchun University of Traditional Chinese Medicine, 2006]. Moreover, an active ingredient, echinocystic acid, is contained in gleditsia sapogenin. It has been disclosed in CN Patent Application No. 03100676.0 with the title of A PREPARATION METHOD OF ECHINOCYSTIC ACID, PHARMACEUTICAL FORMULATIONS COMPRISING THE SAME AND NOVEL MEDICAL USE THEREOF that the echinocystic acid prepared from acidic hydrolysis of gleditsia saponin exhibits an excellent efficacy in treatment of coronary artery disease, angina pectoris, and myocardial ischemia. Presently, there were few reports about gleditsia sapogenin. In addition to echinocystic acid, Chen Xiaolan, et al. reported the chemical structures of gleditsia sapogenins, in which two kinds of sapogenins: 3-hydroxy-12-oleanen-28-oic acid and 3,16-dihydroxy-12-oleanen-28-oic acid, were obtained from pods of Gleditsia sinensis by performing extraction, complete hydrolysis, chromatography etc [Chen Xiaolan, et al. Studies on Structure of Sapogenins from Pod of Gleditsia sinensis, Chinese Traditional and Herbal Drugs, 2001, 32(3)]. However, present researches on gleditsia sapogenin were merely based on single active ingredient. Since extracts from medicinal materials contain a variety of known and unknown ingredients, and gleditsia sapogenin also contains various compounds, how to sufficiently utilize the activities of gleditsia sapogenin is an urgent problem to be resolved.

### SUMMARY OF THE INVENTION

One embodiment of the present invention provides a pharmaceutical composition for treating cardiovascular disorders, and another embodiment of the present invention provides a gleditsiae extract comprising echinocystic acid and oleanolic acid, a preparation method for the same and uses thereof.

The invention provides a pharmaceutical composition for treating cardiovascular disorders, in which the active ingredient consists of:
3-1 part(s) by weight of echinocystic acid and 1 part by weight of oleanolic acid.
   More preferably, the active ingredient consists of:
3-2 parts by weight of echinocystic acid and 1 part by weight of oleanolic acid.
   Even more preferably, the active ingredient consists of:
3 parts by weight of echinocystic acid and 1 part by weight of oleanolic acid.

The echinocystic and oleanolic acids may be obtained from a plant extract or chemical synthetic substances.

The invention also provides use of the pharmaceutical composition in preparation of a medicament for preventing or treating cardiovascular disorders.

Said medicament is the one for preventing or treating coronary artery disease, angina pectoris, myocardial ischemia, myocardial infarction, arrhythmia, hyperlipidemia or atherosclerosis.

The invention provides a gleditsiae extract in which the total weight percentage of echinocystic acid and oleanolic acid is 90.0%-99.9%, and the weight ratio of echinocystic acid to oleanolic acid is 3:1-1:1.

Further preferably, the weight ratio of echinocystic acid to oleanolic acid is 3:1-2:1, and more preferably 3:1.

The invention also provides a method for preparing a gleditsiae extract, comprising the steps of:
a. preparing a medicinal material of *Fructus Gleditsiae Sinensis,* and subjecting the medicinal material to decoction with water, then filtering, and concentrating the filtrate;
b. adding an acid together with water or aqueous ethanol into the concentrated filtrate allowing the concentration of the acid to be 2 mol/L and the content of the optional ethanol to be 1-45%(v/v), stirring and boiling the same for a hydrolysis, then filtering the hydrolyzed solution, washing the obtained filter cake with purified water until the pH of the effluent being above 6, then drying the washed filter cake at 80°C to yield crude gleditsia sapogenin;
c. dissolving the crude gleditsia sapogenin in 95% ethanol while being heated (the amount of 95% ethanol is 15-40 parts by volume(ml) based on the weight(g) of the crude gleditsia sapogenin as one part), adding pharmaceutically acceptable activated carbon therein (the amount of the activated carbon is 1-4% (w/v) based on the volume of 95% ethanol), stirring and boiling the same for 30 min followed by filtering while hot; adjusting pH of the filtrate to 2-6 followed by recovering ethanol therefrom, then cooling and crystallizing, and drying the obtained crystal at 80°C to yield the gleditsiae extract.

The invention also provides uses of said gleditsiae extract in preparation of a medicament for preventing or treating cardiovascular disorders.

Said medicament is the one for preventing or treating coronary artery disease, angina pectoris, myocardial ischemia, myocardial infarction, arrhythmia, hyperlipidemia, or atherosclerosis.

In experiments, the applicant found that echinocystic acid had relatively stronger toxic side-effects during application, and also discovered that not only echinocystic acid showed certain toxicity, but also the gleditsia sapogenin (in which the total content of echinocystic acid and oleanolic acid was about 70%) exhibited certain toxic side-effects. In particular, among six subjects (evenly composed of men and women) who were administered with the gleditsia sapogenin for three times a day and 500mg per time, four subjects (evenly composed of men and women) showed increase in both ALT and AST when they were accumulatively administered for 5-11 times. In order to better exploit the pharmacodynamic performance of echinocystic acid and reduce its toxic side-effects, the applicant found after lots of experimental studies that the active components consisting of echinocystic acid and oleanolic acid in a weight ratio of from 3:1 to 1:1 are able to reduce the toxic side-effects of echinocystic acid in treatment of cardiovascular disorders and thereby to result in wider safety margin for echinocystic acid, thus leading to higher safety and greater potency. At the same time, when an gleditsiae extract is prepared from *Fructus gleditsiae* and optimized by controlling the total content of echinocystic acid and oleanolic acid and adjusting weight ratio between the two acids, the gleditsiae extract comprising echinocystic acid and oleanolic acid in the total content of 90.0% -99.9% with the weight ratio of the former to the latter being 3:1-1:1 was allowed both to treat cardiovascular disorders with significant potency and to exhibit significantly reduced hepatotoxicity. In addition, the production cost thereof was also considerably reduced.

In conclusion, the invention has the following advantages as compared to the prior art:
1. The hepatotoxicity of echinocystic acid is significantly reduced by controlling the ratio between echinocystic acid and oleanolic acid in the pharmaceutical composition, which allows the echinocystic acid to be safer and more effective in treatment of cardiovascular disorders.
2. By controlling weight ratio of echinocystic acid to oleanolic acid in a gleditsiae extract, an optimized gleditsiae extract is obtained which may not only reduce the hepatotoxicity of echinocystic acid, but also reduce the production cost thereof considerably.

### DESCRIPTION OF THE DRAWINGS

Figure 1. The HPLC chromatograms for drugs of: A: echinocystic acid, B: oleanolic acid, C: the gleditsiae extract (echinocystic acid: oleanolic acid = 1:1) prepared in Example 1, D: the gleditsiae extract (echinocystic acid: oleanolic acid = 2.1:1) prepared in Example 2, E: the gleditsiae extract (echinocystic acid: oleanolic acid = 2.4:1) prepared in Example 3, F: the gleditsiae extract (echinocystic acid: oleanolic acid = 3:1) prepared in Example 4.
Figure 2. The pathological picture of the HE-stained aorta in the Z01 group (×100)
Figure 3. The pathological picture of the HE-stained aorta in the Z02 group (×100)
Figure 4. The pathological picture of the HE-stained aorta in the Lipitor group (×100)
Figure 5. The pathological picture of the HE-stained aorta in the Lipanthyl group (×100)
Figure 6. The pathological picture of the HE-stained aorta in the model group (×100)
Figure 7. The pathological picture of the HE-stained aorta in the blank group (×100)

### DETAILED DISCRIPTION OF THE INVETION

### Example 1 Preparation of a gleditsiae extract of the present invention

600 g of the medicinal material of the Chinese honeylocust fruit was weighed out, cleaved into short segments of about 1 cm, and decocted twice with 10 times weight of water, with the first decoction for 1.5 h and the second decoction for 1 h. The filtrates from the first and second decoctions were pooled and concentrated to the relative density of 1.05 (at room temperature) under a reduced pressure. Appropriate amounts of hydrochloric acid, as well as water and ethanol were added into the concentrated solution, so that the resulting solution has a volume to be four times larger than that of the concentrated solution, and has the concentration of the acid to be 2 mol/L and the ethanol content to be 45% (v/v). The resulting solution was heated, stirred and boiled for 3 h to hydrolysis, and then filtered. The filter cake was washed with purified water until the pH of the effluent being above 6, and then dried at 80°C to yield 15.5 g of crude gleditsiae extract. The crude gleditsiae extract was added into 230 ml of ethanol, dissolved by heating, followed by addition of 2.3 g of pharmaceutically acceptable activated carbon, stirred and boiled for 30 min, then filtered while hot. The filtrate was subjected to adjustment of pH value to 2-3, then after standing for 24 h at room temperature, filtered. The filter cake was washed with purified water until the pH of the effluent being above 6, and dried at 80°C to give 6.3 g of gleditsiae extract. The total content of echinocystic acid and oleanolic acid in the obtained gleditsiae extract was determined by high performance liquid chromatography to be 97.78%, in which the ratio of echinocystic acid to oleanolic acid was 1:1.

### Example 2 Preparation of a gleditsiae extract of the present invention

800 g of the medicinal material of the Chinese honeylocust fruit was weighed out, cleaved into short segments of about 1 cm, and decocted twice with 8 times weight of water, with the first decoction for 1.5 h and the second decoction for 1 h. The filtrates from the first and second decoctions were pooled and concentrated to the relative density of 1.10 (at room temperature) under a reduced pressure. Appropriate amounts of hydrochloric acid and water were added into the concentrated solution with the volume of the resulting solution being four times larger than that of the concentrated solution and the concentration of the acid to be 2 mol/L .The resulting solution was heated, stirred and boiled for 3 h to hydrolysis, and then filtered. The filter cake was washed with purified water until the pH of the effluent being above 6, and dried at 80°C to yield 61.2 g of crude gleditsiae extract. The crude gleditsiae extract was added into 2450 ml of ethanol, dissolved by heating, followed by addition of 49.0 g of pharmaceutically acceptable activated carbon, then stirred and boiled for 30 min, and filtered while hot. The filtrate was subjected to adjustment of pH value to 3-4, concentrated to be thick paste-like under a reduced pressure, and cooled, followed by filtration. The filter cake was washed with purified water until the pH of the effluent being above 6, and dried at 80°C to give 8.5 g of gleditsiae extract. The total content of echinocystic acid and oleanolic acid in the obtained gleditsiae extract was determined by high performance liquid chromatography to be 94.11 %, in which the ratio of echinocystic acid to oleanolic acid was 2.1:1.

### Example 3 Preparation of a gleditsiae extract of the present invention

2400 g of the medicinal material the Chinese honeylocust fruit was weighed out, cleaved into short segments of about 1 cm, and decocted twice with 6 times weight of water, with the first decoction for 1.5 h and the second decoction for 1 h. The filtrates from the first and second decoctions were pooled and concentrated to the relative density of 1.20 (at room temperature) under reduced pressure. Appropriate amounts of hydrochloric acid and water were added into the concentrated solution with the volume of the resulting solution being four times larger than that of the concentrated solution and the concentration of the acid to be 2 mol/L. The resulting solution was heated, stirred and boiled for 3 h to hydrolysis, and filtered. The filter cake was washed with purified water until the pH of the effluent being above 6, and dried at 80°C to yield 153.0 g of crude gleditsiae extract. The crude gleditsiae extract was added into 6120 ml of ethanol, dissolved by heating, followed by addition of 122.4 g of pharmaceutically acceptable activated carbon, then stirred and boiled for 30 min, and filtered while hot; the filtrate was subjected to adjustment of pH value to 4-5, concentrated to 850 ml under reduced pressure, and cooled, followed by filtration; the filter cake was washed with purified water until the pH of the effluent being above 6, and dried at 80°C to give 24.1 g of gleditsiae extract. The total content of echinocystic acid and oleanolic acid in the obtained gleditsiae extract was determined by high performance liquid chromatography to be 100.28%, in which the ratio of echinocystic acid to oleanolic acid was 2.4:1.

### Example 4 Preparation of a gleditsiae extract of the present invention

800 g of the medicinal material of the Chinese honeylocust fruit was weighed, cleaved into short segments of about 1 cm, and decocted twice with 8 times weight of water, with the first decoction for 1.5 h and the second decoction for 1 h. The filtrates from the first and second decoctions were pooled and concentrated to the relative density of 1.10 (at room temperature) under reduced pressure. Appropriate amounts of hydrochloric acid and water were added into the concentrated solution with the volume of the resulting solution being four times larger than that of the concentrated solution and the concentration of the acid to be 2 mol/L. The resulting solution was heated, stirred and boiled for 3 h to hydrolysis, and filtered. The filter cake was washed with purified water until the pH of the effluent being above 6, and dried at 80°C to yield 51.0 g of crude gleditsiae extract. The crude gleditsiae extract was added into 2040 ml of ethanol, dissolved by heating, followed by addition of 81.6 g of pharmaceutically acceptable activated carbon, then stirred and boiled for 30 min, and filtered while hot. The filtrate was subjected to adjustment of pH value to 5-6, concentrated to be thick paste-like under reduced pressure, and cooled, followed by filtration. The filter cake was washed with purified water until the pH of the effluent being above 6, and dried at 80°C to give 6.2 g of gleditsiae extract. The total content of echinocystic acid and oleanolic acid in the obtained gleditsiae extract was determined by high performance liquid chromatography to be 96.33%, in which the ratio of echinocystic acid to oleanolic acid was 3:1.

### Assay Methods

Chromatographic condition: Using high performance liquid chromatograph: Dionex U-3000 and chromatographic column: Agilent TC-C18 250x4.6 mm (5µm); using acetonitrile as mobile phase A, and water (of which pH value had been adjusted to 3.7 with phosphoric acid) as mobile phase B, performing a gradient elution according to the table below, with flow rate of 1.0 ml/min, column temperature at 30°C, and detection wavelength of 215 nm.

| Time (min) | Mobile phase A (%) | Mobile phase B (%) |
|---|---|---|
| 0-3 | 80→64 | 20→36 |
| 3-10 | 64 | 36 |
| 10-15 | 64→100 | 36→0 |
| 15-22 | 100 | 0 |
| 22-23 | 100→80 | 0→20 |
| 23-35 | 80 | 20 |

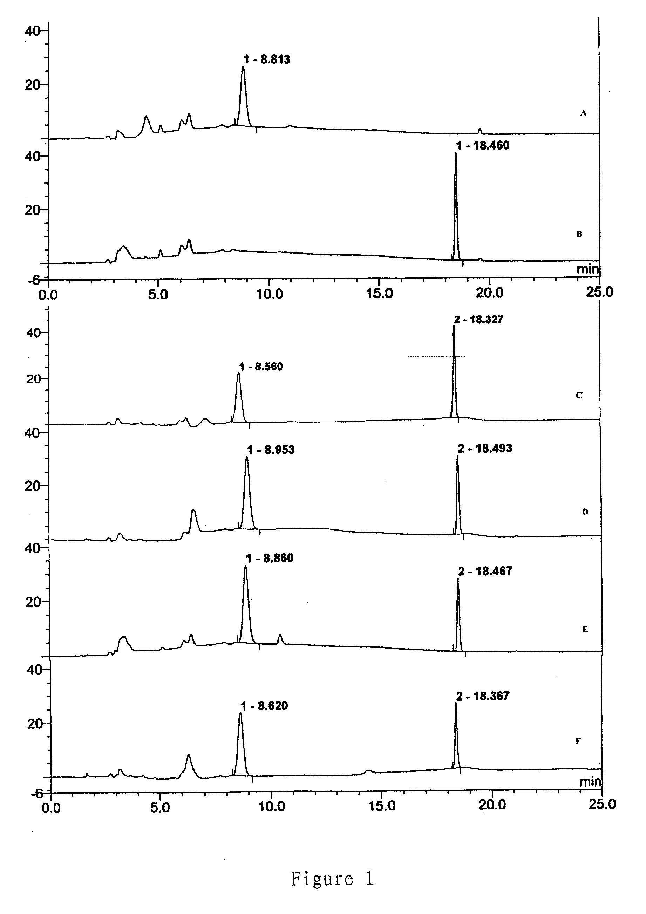

The chromatograms are shown in Fig. 1, and the data are listed as follows: Data of the HPLC chromatograms

| Sample | M-wt of sample (mg) | A-echinocystic acid | Content of echinocystic acid (%) | A-oleanolic acid | Content of oleanolic acid (%) | Content and value (%) | Echinocystic acid:oleanolic acid |
|---|---|---|---|---|---|---|---|
| C | 19.79 | 559.19 | 49.02 | 523.37 | 48.76 | 97.78 | 1.0 |
| D | 21.11 | 780.90 | 64.17 | 342.75 | 29.94 | 94.11 | 2.1 |
| E | 20.63 | 843.94 | 70.97 | 327.96 | 29.31 | 100.28 | 2.4 |
| F | 20.61 | 856.65 | 72.11 | 270.77 | 24.22 | 96.33 | 3.0 |
| Control sample of echinocystic acid | | A-echinocystic (A) acid | C-Control sample of echinocystic acid (µg/ml) | Control sample of oleanolic acid (B) | | A-oleanolic acid | Control sample of oleanolic acid (µg/ml) |
| | | 632.92 | 109.8 | | | 456.67 | 84.2 |

### Preparation of the control samples

The gleditsiae extract prepared above was dissolved with chromatographically pure methanol to its maximum concentration, and loaded onto the GE AKTA basic preparative high performance liquid chromatograph, using chromatographic column Kromasil C18 250x20 mm (5µm), with methanol:water (of which pH value had been adjusted to 3.7 with phosphoric acid) =85:15 as the mobile phase, flow rate 10.0 ml/min, column temperature at 30°C, and detection wavelength at 215nm. Fractions corresponding to the peak of echinocystic acid or oleanolic acid were collected respectively and crystallized to give chinocystic acid (98.5%) or oleanolic acid (99.0%).
Echinocystic acid: white needle crystal, m.p.305 - 307.5°C
IRmaxcm-1: 3572, 3388, 3209, 2641 (caused by stretching vibration of alcohol hydroxyl-OH); 2947, 2925, 2866 (methyl); 1692, 1679 (caused by stretching vibration of C=O in carboxyl group); 1465, 1449, 1368, 1363, 1307 (carboxyl); 1286, 1333, 1235 (hexacarbon ring); 1140, 1204, 1187, 1029, 998, 979, 965, 950, 816 (ethylenic bond); 788, 744
EI-MSm/z (%): 471 (M-H+: 100), 453 (M-H+: 55.0), 441 (M-H+: 80.0), 425 (M-H+: 92.0), 407 (M-H+: 100)
¹HNR4R (DMSO-d6, δ): 0.67-1.95 (41H: -CH₂-, -CH<, -CH₃); 2.21 (1H: -CH₂); 2.88-3.00 (2H: -CH<); 4.32-4.71 (2H: -OH); 5.20 (1 H: -CH<); 12.01 (1H: -COOH)
¹³CNMR (DMSO-d6, δ): 38.17 (C-1), 27.02 (C-2), 76.89 (C-3), 38.42 (C-4), 54.97 (C-5), 18.00 (C-6), 32.70 (C-7), 38.96 (C-8), 46.23 (C-9), 36.63 (C-10), 22.90 (C-11), 121.25 (C-12), 143.98 (C-13), 41.04 (C-14), 35.21 (C-15), 72.98 (C-16), 47.30 (C-17), 40.10 (C-18), 46.38 (C-19), 30.26 (C-20), 34.69 (C-21), 31.50 (C-22), 28.28 (C-23), 16.08 (C-24), 15.25 (C-25) 16.85 (C-26), 26.50 (C-27), 178.24 (C-28), 32.92 (C-29), 24.20 (C-30).
Oleanolic acid: white needle crystal, mp 307°C -308°C, (a) 20D + 73°.
¹HNMR (DMSO-d6, δ): δ ppm: 5.16 (m), 3.36 (t), 1.09 (s, Me), 0.91 (s, Me), 0.84 (s, Me), 0.81 (s, Me), 0.80 (s, Me), 0.71 (s, Me), 0.67 (s, Me).
¹³CNMR (DMSO-d6, δ): 38.5 (C-1), 27.19 (C-2), 76.8 (C-3), 38.22 (C-4), 54.7 (C-5), 18 (C-6), 32.85 (C-7), 39.66 (C-8), 47 (C-9), 37 (C-10), 23.36 (C-11), 121.53 (C-12), 143.82 (C-13), 41.31 (C-14), 25.99 (C-15), 22.86 (C-16), 45.66 (C-17), 41.3 (C-18), 45.68 (C-19), 30.19 (C-20), 33.31 (C-21), 32.41 (C-22), 28.21 (C-23), 15.15 (C-24), 16.1 (C-25), 16.8 (C-26), 26.99 (C-27), 178.6 (C-28), 32.1 (C-29), 22.6 (C-30).

### Example 5 Preparation of a capsule of the present invention

500 g of the gleditsiae extract, 1600 g of lactose, 340 g of starch, and 50 g of sodium carboxymethyl starch were mixed uniformly, granulated with appropriate amount of 70% ethanol, dried, blended with 10 g of magnesium stearate, and the obtained mixture was encapsulated into No. 1 capsule with 250mg per capsule.

### Example 6 Preparation of a tablet of the present drug

400 g of the gleditsiae extract, 800 g of microcrystalline cellulose, 500 g of lactose, 125 g of polyvinylpyrrolidone K30, 125 g of croscarmellose sodium, and 40 g of SiliciiDoxydum were mixed uniformly, granulated with appropriate amount of 50% ethanol, dried, blended with 10 g of magnesium stearate, and pressed into tablets with 200 mg per tablet.

Other kinds of tablets were prepared according to the above procedure using 300.4 g of echinocystic acid (in-house made, purity of 98.5%) and 99.6g of oleanolic acid(in-house made, purity of 99.0%), or 200.5 g of echinocystic acid (in-house made, purity of 98.5%) and 199.5 g oleanolic acid(in-house made, purity of 99.0%) instead of the gleditsiae extract respectively.

### Example 7 Preparation of a pharmaceutical composition of the present invention

300.4 g of echinocystic acid (the above prepared, purity of 98.5%), 99.6 g of oleanolic acid (the above prepared, purity of 99.0%), 1815 g of microcrystalline cellulose, 50 g of sodium dodecylsulfate, 100 g of cross-linked polyvinylpyrrolidone, and 125 g of hydroxypropyl methyl cellulose were mixed uniformly, granulated with appropriate amount of purified water, dried, blended with 10 g of magnesium stearate, and the mixture was encapsulated into No. 1 capsule with 250mg per capsule.

Another kind of capsules were prepared according to the above-mentioned procedure by using 200.5 g of echinocystic acid (the above prepared, purity of 98.5%) and 199.5 g of oleanolic acid (the above prepared, purity99.0%).

Comparative Example: Gleditsia Sapogenin were prepared according to the process for extraction of gleditsia sapogenin described in HAO Yufang, "Preparation of Gleditsia Sapogenin and Study on its Anti-Myocardial Ischemia Efficacy," Master's Degree thesis, Changchun University of Traditional Chinese Medicine, 2006. The dried medicinal material of the Chinese honeylocust fruit was weighed out, cleaved into short segments of about 1 cm, and extracted three times with 75% ethanol under reflux, with the volumes of ethanol used in the three extractions being 6, 5, and 5 times (v/w) of the weight of the crude drug, and for 3, 2, and 1.5 h respectively. The extracts from the three extractions were pooled, concentrated under reduced pressure to have a relative density of 1.20 -1.30 (measured at 50°C), dried (80°C) under reduced pressure to yield gleditsia saponin. The resulting gleditsia saponin was hydrolyzed in an ethanol solution (the concentration of ethanol is 45%) containing 2 mol/L hydrochloric acid with a volume of 12 times (v/w) of the weight of the gleditsia saponin at 100°C for 3 h. The hydrolysis product was filtered and the hydrolyzate was discarded. The filter cake was washed with deionized water until neutral, and dried to give crude gleditsia sapogenin. Then, the crude gleditsia sapogenin was dissolved in 95% ethanol with a volume of 40 times (v/w) of the weight of the crude gleditsia sapogenin, and the solution was heated to boil followed by addition of 1% of pharmaceutically acceptable activated carbon (based on volume of the solution) therein, then filtered while hot. The filtrate was concentrated under reduced pressure to recover ethanol therefrom, and the solid was dried (at 80°C) to obtain the gleditsia sapogenin. Totally three batches were prepared and the assay results were shown in Table 1.

**Table 1 Assay results for the three batches from the comparative process**

| Lot Number | Yield of Sapogenins (%) | Content of echinocystic acid (%) | Content of oleanolic acid (%) | Total content (%) |
|---|---|---|---|---|
| 1 | 2.17 | 52.40 | 21.46 | 73.86 |
| 2 | 2.12 | 56.56 | 20.40 | 76.96 |
| 3 | 2.46 | 57.64 | 18.83 | 76.47 |

The average yield of sapogenins was 2.25%, in which the average content of echinocystic acid was 55.53%, the average content of oleanolic acid was 20.23%, and average total content of toechinocystic acid and oleanolic acid was 75.76%.

The following disadvantages of the comparative process are inherent:
a. A large amount of ethanol is used in the extraction and hydrolysis procedures, which results in high cost of production.
b. The drying procedure under reduced pressure is performed on the mixture of the stock solution, which is unsafe and also unsuitable for industrialized production.
c. The prepared sapogenins comprise more than 20% other ingredients and impurities.
d. The obtained sapogenins are grey-white powder, and turn dark brown when exposed to solvents such as water, alcohol and the like during formulation, which is disadvantageous to development of novel dosage forms with increased compliance of patients, such as orally disintegrating tablets, sustained- and/or controlled-formulations, etc.

As compared to the comparative process, the present process of preparing the gleditsiae extract can control the total content of echinocystic acid and oleanolic acid and the weight ratio therebetween by controlling the conditions in the extraction process, such as pH value of hydrolysis, the quantity of pharmaceutically acceptable activated carbon used, etc. The present process of preparing the gleditsiae extract has the following advantages:
a. In the extraction procedure, water as extraction solvent is used instead of alcohol and the hydrolysis procedure may be performed in absence of alcohol, which reduces the cost of production considerably.
b. Direct hydrolysis is used instead of the drying procedure under reduced pressure, which decreases the energy consumption and increases the production efficiency, and the acidic hydrolysis in absence of alcohol is utilized, which reduces the cost of production.
   Industrialized production of gleditsiae extract is enabled by the two advantages above, i.e. the increased production efficiency and the reduced cost of production, as well as the enhanced safety in the manufacture, which allows the gleditsiae extract to be further developed into medicaments favoring more patents.
c. The gleditsiae extract of the present invention is white or off-white crystal, in which total content of the echinocystic acid and oleanolic acid is 90% or above, indicating the significantly improved quality, highly pure gleditsiae extract is more favorable to establish the extract standard and also to develop various new dosage forms according to the need of market competition.
d. The improved process significantly increases the quality of the gleditsiae extract and also enhances the stability of and controllability over the quality thereof, which is more favorable to development of medicament.

Other beneficial effects of the present invention will be demonstrated by the following toxicological and pharmacodynamic tests.

### Test Example 1 Comparison of toxicological tests between the present pharmaceutical composition and the echinocystic acid

### Materials and methods

Animals: Conventional, healthy adult Beagle canines, evenly composed of males and females, 6-8 month-old, body weight of 9 - 11 kg.

Medicaments: Z01 (echinocystic acid:oleanolic acid = 3:1, prepared by the method in Example 4, in which the total content of the echinocystic acid and the oleanolic acid is 99%), in-house-made off-white tablet, Lot number: 090205; Z02 (echinocystic acid:oleanolic acid = 1:1, prepared by the method in Example 1, in which the amount of echinocystic acid and oleanolic acid accounts to 94.2% of the amount of the extract), in-house-made off-white tablet, Lot number: 090206; in-house-made echinocystic acid EA (containing 98.5% of echinocystic acid), pure-white powder, Lot number: 090107; physiological saline, colorless clarified liquid, provided by Chengdu QingShan LiKang Pharmarceutical co., Ltd., Lot number: A0803025; Alanine Transarninase (ALT) Kit, provided by Sichuan MiKe Science & Technology Co., Ltd., Lot number: 1007071.

Major instruments: a fully automated blood biochemistry analyzer (Roche Integra 400 Plus, Switzerland), a fully automated hematology analyzer (Abbott CELL-DYN 3700SL, USA), a fully automated blood coagulation analyzer (SYSMEX CA-500, Japan), a four-channel physiological recording system (RM6240, Chengdu Instrument Factory, China), electronic balance, etc.

Procedure: The normal Beagle canines were subjected to quanrantine for two weeks and a dog with abnormal parameters was rejected. The selected dogs were assigned randomly into ten groups: blank control group, low dose Z01 group (the dosage of echinocystic acid, 50 mg/kg), medium dose Z01group (the dosage of echinocystic acid, 150 mg/kg), high dose Z01 group (the dosage of echinocystic acid, 250 mg/kg), Z02 low dose group (the dosage of echinocystic acid, 50 mg/kg), medium dose Z02 group (the dosage of echinocystic acid, 150 mg/kg), high dose Z02 group (echinocystic acid is 250 mg/kg), low dose EA group (50mg/kg), medium dose EA group (150mg/kg), high dose EA group (250mg/kg), with 8 animals per group evenly composed of males and females. All of the dogs in individual groups were intragastrically administered at a volume of 10 ml/kg, once per day and successively for 21 days. Prior to and at Day 21 after the administration, samples of urine excreted naturally in the morning and blood from forelimb vein were collected respectively for urine routine, blood routine, and coagulation-time determination, meanwhile the lead II electrocardiogram was traced. Prior to the administration and at Day 4, Day 7, Day 14, and Day 21 after the first administration, blood samples were collected from the canine forelimb vein and assayed on the biochemical indicator associated with liver function. In twenty-four hours after the final administration, the animals in each group were dissected, autopsied systematically and observed with naked eyes. The parenchymal organs were weighed, subjected to calculation of organ coefficients, and examined histopathologically. The data in the experiment were treated with the statistical software package SPSS 11.5, and indicated as mean ±standard deviation( *X̅* ±*S.D*.). The comparisons among multiple groups were tested by One-way analysis of variance, and the comparisons between two groups were tested by t-test. Pathologic results were descriptively discussed. The results in details are shown in Table 1.

### Results:

### 1. Biochemical testing and results of ALT in the tested animals

The results in Table 1 indicated that for groups administrated with echinocystic acid as the single component, all levels of alanine transaminase in the blood from three groups administered at doses of 50, 150, and 250 mg/kg increased at the 7th day after administration and sustained until the 21st day after administration, and degree of increase in the level of alanine transaminase is dose-dependent on echinocystic acid. After administration, the level of alanine transaminase in blood from the two groups administered at doses of 150 and 250 mg/kg were above the normal range for canine. Regarding Z01 groups (weight ratio of echinocystic acid to oleanolic acid being 3:1), the levels of alanine transaminase (ALT) in the groups administered with echinocystic acid at doses of 50 and 150 mg/kg were found to be not significantly affected after administration; while that in the group administered with echinocystic acid at a dose of 250 mg/kg showed an increasing trend at the 7th day after administration, and reached the highest value at the 14th day after administration (above the normal range for canine), but slightly decreased at the 21st day after administration. The levels of alanine transaminase after administration in the three Z02 groups (weight ratio of echinocystic acid to oleanolic acid being 1:1) administered with echinocystic acid at doses of 50, 150, and 250 mg/kg were not significantly affected.

### 2. Other testing and results

During administration, only a few animals presented symptoms, such as vomiting, diarrhea, etc. No other abnormal sign was observed in each animal and the body weights of the animals were not significantly changed as compared with that prior to administration. All of testing results from urine routine, blood routine, and coagulation-time determination, lead II electrocardiogram and various biochemical indicators except for ALT at the every monitoring-points were not significantly changed as compared to those prior to administration and those of the blank control group, these results are meaningless in statistical significance.

3. Conclusion: The dose levels set for the three medicaments in the experiment were based on the content of echinocystic acid, that is, the contents of echinocystic acid in the low-, medium-, and high-dose groups were 50, 150, and 250 mg/kg, respectively. The result of such experiment indicates that echinocystic acid enables an increase in ALT level in blood and shows dose-effect relationship with the ALT level, that is, echinocystic acid can cause certain hepatotoxicity. However, the toxic reaction of echinocystic acid on the liver can be considerably reduced by controlling the ratio of echinocystic acid to oleanolic acid in the pharmaceutical composition in a range of 3:1 - 1: 1.

**Table 1 Effects on biochemical index ALT in blood of canine in long-term toxicity test ( X ±S.D.)**

| Group | Echinocystic acid content (mg/kg) | Prior to administration | ALT at different time-points after administration (U/L) | | | |
|---|---|---|---|---|---|---|
| | | | Day 4 | Day 7 | Day 14 | Day 21 |
| Z01 low | 50 | 39.6±8.2 | 40.7±7.6 | 41.2±7.2 | 38.7±5.2 | 41.2±4.2 |
| Z01 medium | 150 | 38.0±6.2 | 41.9±8.1 | 39.2±5.5 | 41.3±8.4 | 43.2±5.9 |
| Z01 high | 250 | 41.8±4.6 | 41.5±9.8 | 53.8±12.1* | 88.3±20.1* | 56.7±12.8* |
| 202 low | 50 | 38.2±4.7 | 33.9±3.6 | 39.4±3.76 | 37.5±8.9 | 44.9±4.9 |
| Z02 medium | 150 | 39.7±4.3 | 42.8±5.7 | 38.9±3.8 | 40.3±6.9 | 38.4±3.1 |
| Z02 high | 250 | 38.5±4.9 | 39.5±4.6 | 42.6±14.2 | 45.3±12.2 | 43.5±9.5 |
| EA low | 50 | 38.8±5.8 | 41.4±9.3 | 60.3±11.6* | 58.3±9.1* | 69.7±6.2* |
| EA medium | 150 | 40.3±6.2 | 42.6±8.9 | 70.6±22.3** | 121.3±19.3** | 124.5±35.3** |
| EAhigh | 250 | 37.3±5.2 | 45.4±9.9 | 146.5±49.0** | 167.5±43.3** | 151.3±86.6** |
| Blank | - | 38.7±4.3 | 41.5±7.7 | 38.6±7.2 | 40.5±5.1 | 36.3±2.7 |

| | | | | | | |
|---|---|---|---|---|---|---|
| Note: *Difference of the mean is of statistical significance as compared to that prior to administration (P < 0.05); **Difference of the mean is of statistical significance as compared to that prior to administration (P < 0.01). | | | | | | |

### Test Example 2 Effects of the pharmaceutical composition of the invention on myocardial infarction caused by ligating coronary artery of an anesthetized canine

### Materials and methods

Animal: healthy, adult hybrid canines, both males and females used, body weight of 12 -16 kg.
Medicaments: Z01 (echinocystic acid:oleanolic acid = 3:1, in which the total content of the echinocystic acid and the oleanolic acid is 99.0%), in-house-made off-white tablet, Lot number: 090205; Z02 (echinocystic acid:oleanolic acid = 1:1, in which the total content of the echinocystic acid and the oleanolic acid is 94.2%), in-house-made off-white tablet, Lot number: 090206; Compound Tablet of Red Sage Root (abbreviated hereinafter as "CTRSR"), brown tablet, manufactured by Sichuan Shuzhong Pharmaceutical Co., Ltd., Lot number: 080202; sodium pentobarbital, white or off-white powder, manufactured by Sigma Corporation, USA, Lot number: P3761; sodium heparin, colorless or yellowish clear liquid, manufactured by Qilu Pharmaceutical. Co., Ltd., Lot number: P20080227152652765; nitrotetrazolium blue chloride, from Shanghai Jingchun Reagent Co.,Ltd, yellow powder, Lot number: 1124927.

Instruments: HX-300 respirator for animal (Chengdu TME Technology Co, Ltd, China), and a fully automated biochemistry analyzer (Roche Integra 400 Plus, Switzerland).

Procedure: Experimental canines were subjected to acclimation feeding and assigned randomly into seven groups: the model group, the negative control group(the sham operation group), the positive control group(Compound Tablet of Red Sage Root of 25 mg/kg), high dose Z01 group (44.4 mg/kg), low dose Z01 group(22.2 mg/kg), high dose Z02 group (70 mg/kg), low dose Z02 group (35 mg/kg), with 6 animals per group. Each of the Experimental animals was anesthetized by intravenous injection of 3% sodium pentobarbital (30 mg/kg). Operation on neck and endotracheal intubation were performed, followed by linkage to the artificial respirator. The carotid in one side was separated and connected to a pressure transducer. The chest was open. The cardiac pericardium was scissored longitudinally and fixed by purse-string suture to hold the heart. The left circumflex coronary artery and root of the aorta were separated. 3 ml of blood was sampled and tested to determinate the activities of phosphoric acid creatine kinase (CK), lactate dehydrogenase(LDH), and glutamic oxaloacetic transaminase (AST) prior to ligation. Then, the coronary artery was ligated (for the sham operation group, no ligation). Fifteen minutes later, the individual indicators were determined. At the same time, animals in the test groups were administered with the medicaments through the duodenum and animals in the model and sham operation groups were administered with equivalent volume of physiological saline through the same route. The individual indicators were determined respectively at 30 min, 60 min, 90 min, 120 min, and 180 min after administration. Finally, 3 ml of blood was sampled and tested to determinate the activities of CK, LDH, and AST after ligation or administration. Then, the animal was sacrificed. The heart was removed, washed with water to remove residue blood, wiped with filter paper to dry moisture, and weighed. After that, the ventricle was sliced into thin sections with thickness of about 1.0 cm. The thin sections were placed into a solution of 2% Red tetrazoline (TTC), incubated at 37°C for 15 min to be stained, and took out of the solution. The unstained myocardium was separated from the thin section and weighed, thus the percentage of infarcted parts against the ventricular was calculated. The data in the experiment were treated statistically with the software SPSS 11.5. Both comparisons of the mean within a group and between groups were tested by One-way analysis of variance with results indicated by x ± s.

### Results:

### 1. Effects on areas of myocardial infarction in canine

The results in Table 2, which are determined areas of myocardial infarction in canines, show that areas of myocardial infarction in the infracted canines from the treated groups and the CTRSR group were significantly reduced in comparison with those from the model group (P < 0.01), and as compared to CTRSR group, there were no significant difference among the administrated groups.

**Table 2 Effects of the pharmaceutical composition of the invention on areas of myocardial infarction in canine myocardial infarction model resulting from ligation of coronary artery ( x ± s , n=6)**

| Group | Dosage (mg/kg) | Whole heart weight (g) | Ventricular weight (g) | Infarcted myocardium (wt%) | Infarcted myocardium/whole heart (wt%) | Infarcted myocardum/ventrtcular (wt%) |
|---|---|---|---|---|---|---|
| Z01 high | 44.4 | 97.8±5.7 | 68.4±8.9 | 8.3±1.1** | 8.5±1.3** | 12.1±1.8** |
| Z01 low | 22.2 | 99.5±7.3 | 76.6 ± 7.1 | 10.8 ± 3.2* | 10.9±3.6* | 14.1±2.7** |
| Z02 high | 70 | 98.6±6.1 | 76.3 ± 5.8 | 9.7±3.2* | 9.8±2.5** | 12.7±2.4** |
| Z02 low | 35 | 97.7±5.9 | 75.2±6.6 | 9.7±0.9** | 9.9±0.7** | 12.9±1.2** |
| Positive control | 25 | 102.4±6.8 | 77.4±9.4 | 9.6±4.2* | 9.4±3.6** | 12.4±5.5** |
| Model | - | 100.3±15.2 | 73.5±8.0 | 20.1 ±3.9 | 20.0±1.9 | 27.0±2.3 |
| Sham operation | - | 98.6±7.4 | 75.4±6.6 | 0.7±0.5** | 0.7±3.0** | 0.9±4.4** |

| | | | | | | |
|---|---|---|---|---|---|---|
| Note: in comparison with the model group, *: P < 0.05; **: P < 0.01 | | | | | | |

### 2. Effects on myocardial enzymes in canine

The results in Table 3, which are determined activities of myocardial enzymes, show that the activities of phosphoric acid creatine kinase (CK) in the canines with myocardial ischemia from each of the administrated groups were significantly decreased in comparison with those from the model group, and had no significant difference as compared to CTRSR group. The activities of lactate dehydrogenase (LDH) in the canines with myocardial ischemia from the Z01 high and Z02 high groups were significantly decreased in comparison with those from the model group; the activities from each of the administrated groups had no significant difference as compared to CTRSR group.

**Table 3 Effects of the pharmaceutical composition of the invention on CK and LDH in canine with myocardial ischemia ( x ± s, n=6).**

| Group | Dosage (mg/kg) | CK(U/L) | | LDH(U/L) | |
|---|---|---|---|---|---|
| | | Prior to ligation | 3 h after ligation | Prior to ligation | 3 h after ligation |
| Z01 high | 44.4 | 214.3±24.4 | 318.7±18.6** | 104.4±10.6 | 160.5±14.2* |
| Z01 low | 22.2 | 225.6±21.3 | 335.7±17.6** | 106.7±13.6 | 174.5±12.6 |
| Z02 high | 70 | 208.7±23.1 | 315.6±17.1** | 105.6±14.7 | 158.4±8.0* |
| Z02 low | 35 | 209.2±18.2 | 346.2±19.7** | 109.1±9.7 | 178.8±12.4 |
| Positive control | 25 | 220.3±20.4 | 322.0±14.1** | 105.6±11.3 | 185.6±10.1 |
| Model | - | 206.4±23.0 | 927.2±36.8 | 103.4±10.1 | 212.6±16.2 |
| Sham operation | - | 212.4±17.0 | 209.2±19.8** | 107.7±13.3 | 105.4±12.3** |

| | | | | | |
|---|---|---|---|---|---|
| Note: in comparison with the model group, *: P < 0.05; **: P < 0.01 | | | | | |

The above results suggest that the two types of the pharmaceutical compositions of the present invention have the significant improvement on myocardial infarction in canine.

### Test Example 3 Effects of the pharmaceutical composition of the invention on hemodynamics after myocardial ischemia of an anesthetized canine

### Materials and methods

Animal: healthy, adult hybrid canines, both males and females used, body weight of 12-16 kg.

Medicament: Z01 (echinocystic acid:oleanolic acid = 3:1, in which the total content of the echinocystic acid and the oleanolic acid is 99.0%), in-house-made off-white tablet, Lot number: 090205; Z02 (echinocystic acid:oleanolic acid = 1:1, in which the total content of the echinocystic acid and the oleanolic acid is 94.2%), in-house-made off-white tablet, Lot number: 090206; CTRSR, brown tablet, manufactured by Sichuan Shuzhong Pharmaceutical Co., Ltd., Lot number: 080202; sodium pentobarbital, manufactured by Sigma Corporation,USA , white or off-white powder, Lot number: P3761.

Instruments: four-channel physiological recording system (RM-6000) from Nihon Kohden Co., electromagnetic flow meter 5.0mm, 1.0mm from Nihon Kohden Co., An automatic blood gas analyzer (AVLCOMPACT-3, Sweden).

Procedure: Experimental canines were subjected to acclimation feeding and assigned randomly into seven groups: the model group, the negative control group(the sham operation group), the positive control group(Compound Tablet of Red Sage Root of 25 mg/kg), high dose Z01 group (44.4 mg/kg), low dose Z01 group(22.2 mg/kg), high dose Z02 group (70 mg/kg), low dose Z02 group (35 mg/kg), with 6 animals per group. Experimental animals were anesthetized by intravenous injection of 3% sodium pentobarbital (30 mg/kg). Operation on neck and endotracheal intubation were performed, followed by linkage to the artificial respirator. A fragment of artery was carefully separated below the second major branch of the anterior descending coronary artery and threaded for ready use, and then the root of ascending aorta was also separated and used for immobilizing the electromagnetic flow meter to record the cardiac output. The left ventricular apex was intubated a cardiac catheter through which the left ventricular pressure and differential (±dp/dtmax) was recorded by a pressure sensor. Electrodes for electrocardiogram were fixed on the limbs of the animal to monitor and record the electrocardiogram. After the normal blood pressure, electrocardiogram, cardiac output, left intraventricular pressure and ±dp/dtmax were recorded, ligation was performed at the free terminus of the left anterior descending of the coronary artery to induce myocardial ischemia. At the same time, animals were administered with drugs according to the assigned groups through the duodenum; and animals in the model and sham operation groups were administered with equivalent volume of physiological saline through the same route. Blood samples obtained respectively from carotid and coronary sinus were analyzed for blood oxygen content using the automatic blood gas analyzer. The data in the experiment were treated statistically with the software SPSS 11.5. Both the comparisons of means within a group and between groups were tested by One-way analysis of variance with results being indicated by x ± s.

### Results:

### 1. Effects on myocardial oxygen consumption in canine suffering from myocardial infarction

In Table 4, the calculated results of the myocardial oxygen consumption (V02) in each of the groups show that the myocardial oxygen consumptions in every dose groups are significantly decreased in 1-4 h after ligation in comparison with that in the model group, and have no significant difference as compared to that in the CTRSR group.

**Table 4 Effects of the pharmaceutical composition of the invention on myocardial oxygen consumption in canine suffering from myocardial infarction ( x ± s, n = 6).**

| Group | Dosage (ml/kg) | The values at the time-points after ligation (ml/min)/percentage (%) | | | | | |
|---|---|---|---|---|---|---|---|
| | | Prior to ligation | 15 min | 30 min | 1 h | 2h | 4h |
| Z01 high | 44.4 | 50.4±5.4 | 53.8±4.2 | 50.6±2.4 | 47.7±1.2* | 45.6±1.1** | 45.7±1.0** |
| Z01 low | 22.2 | 51.5±2.3 | 53.4±2.1 | 49.9±8 | 48.7±1.5* | 46.8±.2** | 47.1 ±1.4* |
| Z02 High | 70 | 50.6±4.4 | 51.9±3.9 | 49.7±2.0 | 47.8±1.5* | 46.6±0.9** | 45.0±1.6** |
| Z02 low | 35 | 50.5±2.3 | 52.6±2.5 | 49.5±1.1 | 48.5±1.6* | 46.7±1.9** | 46.9±1.4* |
| Positive control | 25 | 51.8±1.7 | 53.1±1.8 | 50.3±1.6 | 49.0±1.2* | 47.0±0.9* | 46.7±1.0* |
| Model | - | 50.8±3.1 | 53.0±3.6 | 55.9±0.9 | 56.5±2.5 | 56.2±0.9 | 55.9±1.8 |
| Sham operation | - | 51.7±2.0 | 50.2±3.1 | 51.8±4.4 | 50.6±2.3 | 50.2±3.6 | 50.1±4.0 |

| | | | | | | | |
|---|---|---|---|---|---|---|---|
| Note: in comparison with the model group, *: P < 0.05; **: P < 0.01 | | | | | | | |

### 2. Effects on cardiac output in canine suffering from myocardial infarction

In Table 5, the determined results of cardiac output (AF) show that the cardiac outputs in each of the dose groups are of significant difference in 30 min - 4 h after administration in comparison with that in the model group (P < 0.05, P < 0.01), and have no significant difference as compared to that in the CTRSR group.

**Table 5 Effects of the pharmaceutical composition of the invention on cardiac output in canine suffering from myocardial infarction ( x ± s, n = 6).**

| Group | Dosage (ml/mg) | Change rate of the values (L/beat/m2) at the time-points after ligation(%) | | | | | |
|---|---|---|---|---|---|---|---|
| | | Prior to ligation | 15 min | 30 min | 1 h | 2h | 4h |
| Z01 high | 44.4 | 118.3±74.8 | 167.6±133.3 | 178.9±183.0* | 247.6±274.6* | 201.7±258.4* | 189.5±190.5** |
| Z01 low | 22.2 | 101.9±24.0 | 164.9±20.5 | 190.3±32.4* | 188.1±43.8* | 182.8±37.4* | 181.3±42.2** |
| Z02High | 70 | 103.1±9.2 | 171.3±28.9 | 184.1±30.2* | 183.3±34.9* | 192.6±40.1* | 198.2±42.4** |
| Z02 low | 35 | 98.7±11.5 | 167.8±9.1 | 184.5±13.9* | 188.7±26.2* | 186.2±51.0* | 184.3±38.2** |
| Positive control | 25 | 115.8±36.4 | 141.6±31.0 | 170.3±60.4* | 189.1±42.0* | 188.5±44.1* | 185.9±38.2** |
| Model | - | 121.9±51.7 | 132.3±57.7 | 138.8±79.5 | 129.3±98.3 | 144.5±72.8 | 115.5±43.4 |
| Sham operation | - | 135.7±109.9 | 137.9±94.7 | 148.0±149.7 | 119.2±69.7 | 136.7±44.9 | 146.4±38.3 |

| | | | | | | | |
|---|---|---|---|---|---|---|---|
| Note: in comparison with the model group, *: P < 0.05; **: P < 0.01 | | | | | | | |

### 3. Effect on cardiac work in canine suffering from myocardial infarction

In Table 6, the calculated results of cardiac work (MW) show that the cardiac works in the dose groups and the CTRSR group are significantly decreased in 30 min after administration, and the cardiac works in the dose groups are significantly decreased in 30 min - 4 h after administration (P < 0.05) as compared to that in the model group, in which the cardiac works in the high dose Z01 group and high dose Z02 group are more significantly decreased at the time-points of 1 h and 2 h after administration, respectively (P < 0.01); and there is no significant difference between the dose groups and the CTRSR group.

**Table 6 Effects of the pharmaceutical composition of the invention on cardiac work in canine suffering from myocardial infarction ( x ± s, n = 6).**

| Group | Dosage (ml/kg) | Change rate of the value (L/beat/m2) at the time-point after ligation(%) | | | | | |
|---|---|---|---|---|---|---|---|
| | | Prior to ligation | 15 min | 30 min | 1 h | 2h | 4h |
| Z01 high | 44.4 | 2.82±0.47 | 2.16±0.14 | 2,08±0.16* | 2.13±0.20** | 2.06±0.24** | 2.01±0.22* |
| Z01 low | 22.2 | 2.85±0.35 | 2.14±0.29 | 2.00±0.11* | 2.04±0.24** | 2,01±0.13* | 1.96±0.34* |
| Z02 high | 70 | 2.84±0.41 | 2.17±0.29 | 2.14±0.12* | 2.18±0.29** | 2.06±0.12** | 2.04±0.25* |
| Z02 low | 35 | 2.77±0.39 | 2.08±0.16 | 2.04±0.18* | 1.99±0.22* | 1.96±0.14* | 1.92±0.20* |
| Positive control | 25 | 2.74±0.29 | 2.09±0.22 | 2.06±0.23* | 2.11±0.32* | 2.01±0.31* | 1.98±0.29* |
| Model | - | 2.81±0.44 | 1.84±0.27 | 1.59±0.34 | 1.54±0.21 | 1.56±0.29 | 1.59±0.17 |
| Sham operation | - | Z.95±0.35 | 2.84±0.39* | 2.87±0.47** | 2.86±0.50** | 2.85±0.33** | 2.86±0.36** |

| | | | | | | | |
|---|---|---|---|---|---|---|---|
| Note: in comparison with the model group, *: P < 0.05; **: P < 0.01 | | | | | | | |

In a word, the two types of the pharmaceutical compositions show an anti-myocardial infarction effect, the mechanism of which is reduction of myocardial oxygen consumption by improving myocardial mechanics, as well as stabilization and protection of the cellular membrane of the ischemic myocardial cell to some extent. The compositions may be developed into new drugs for treating myocardial infarction, angina pectoris and related disorders.

Test Example 4 Effects of the pharmaceutical composition of the invention on rat myocardial ischemia caused by pituitrin (PIT).

### Materials and methods

Animal: SD rats in clean grade, 5-6 week-old, body weight of 200-250 g, both males and females used.

Medicament: X01 (echinocystic acid:oleanolic acid = 3:1, prepared by the method in Example 4, in which the total content of the echinocystic acid and the oleanolic acid is 92.8%), in-house-made off-white tablet, Lot number: 090309; X02 (echinocystic acid:oleanolic acid =1:1, prepared by the method in Example 1, in which the total content of the echinocystic acid and the oleanolic acid is 98.8%), in-house-made off-white tablet, Lot number: 090310; pituitrin (PIT): Lot number: 080315, manufactured by Bengbu Hongye Biochemical pharmaceuticals Factory, sodium pentobarbital: Lot number: F20020915; imported and distributed by *Sinopharm Chemical* Reagent (Shanghai, China) Co., Ltd; Tongxinluo capsule, Lot number: 080749, manufactured by Shijiazhuang Yiling Pharmaceutical Co., Ltd.

Instruments: ASB240U Biosignal Acquisiton System, Chengdu Aosheng Electronics Co., Ltd; KQ5200B Ultrasonic Cleaner , Kunshan Ultrasonic Instrument Co. Ltd.; Milli-Q Gradient A10 Pure Water Device: USA Millipore Co.; AB 104-N Electronic Balance: METTLER TOLEDO Co.

Procedure: 112 SD rats sensitive to pituitrin (PIT) and having normal ECG were screened, who were 5 - 6 weeks old and had body weight of 200-250 g. They were assigned randomly into seven groups: the model group, the blank control group, the positive control group (Tongxinluo capsule of 360 mg/kg), X01 high dose group (69.5 mg/kg), X01 low dose group (34.7 mg/kg), X02 high dose group (97.7 mg/kg), X02 low dose group (48.8 mg/kg), with 15 animals per group evenly composed of males and females. The animals in each of the groups were intragastrically administered with the corresponding drugs in a volume of 10 ml/kg, for 7 successive days. A rat was injected intraperitoneally with 1% sodium pentobarbital (30 mg/kg) in 100 min after the final administration. When anesthetized, the animal was immobilized onto a frog board and subjected to trace of normal electrocardiogram over 2 min with Lead II. Then, PIT (1.5 IU/kg) was injected slowly over 20 s into the rat through its tail vein. The changes in the electrocardiogram were traced in 15 min after PIT injection. The data from the ST segment were collected at the time-points of 10 s, 30 s, 1min, 2min, 4min, 6min, 8min, 10 min, 13min, and 15min. ST segment shifts at the various time-points after modeling were compared.

### Results:

It is known from analyzing the sum of ST segment shift in Table 7 that both the high doses and low doses of the two samples can antagonize ST segment shift in some extent, and the antagonisms in the X01 high dose group, the X02 high and low dose groups are most significant (P <0.05 or P <0.01). According to the results of ST segment shift in the myocardial ischemia model at various time-points, X02 exhibits a significant effect of anti-myocardial ischemia in 10 sec, 10 min, 13min, and 15 min after injection of PIT. The results demonstrate that the two samples of the pharmaceutical compositions X01 and X02 both have significant effects of anti-myocardial ischemia and can be used in early and recovery periods of coronary heart attack.

**Table 7 Effects of the pharmaceutical composition of the invention on ST segment of the electrocardiogram in rat with an acute myocardial ischemia caused bv PIT ( x ± s, n = 15).**

| Group | Dosage (mg/kg) | ΔST(mV) | | | | | | | | | | |
|---|---|---|---|---|---|---|---|---|---|---|---|---|
| | | 10 sec | 30 sec | 1 min | 2 min | 4 min | 6 min | 8 min | 10min | 13 min | 15 min | Σ |
| X01 high | 69.5 | 0.054± 0.050** | 0.057± 0.057 | 0.059± 0.074 | 0.056± 0.048 | 0.060± 0.055* | 0.081± 0.065 | 0.054± 0.058 | 0.039± 0.043 | 0.038± 0.031* | 0.040± 0.028* | 0.569± 0.325* |
| X01 low | 34.7 | 0.121± 0.070 | 0.080± 0.043* | 0.079± 0.078 | 0.092± 0.087 | 0.107± 0.093 | 0.074± 0.065 | 0.057± 0.045 | 0.046± 0.030 | 0.043± 0.019 | 0.043± 0.023 | 0.743± 0.399 |
| X02 high | 97.7 | 0.074± 0.068* | 0.049± 0.045 | 0.045± 0.032 | 0.078± 0.051 | 0.085± 0.049 | 0.079± 0.073 | 0.041± 0.030 | 0.022± 0.017** | 0.019± 0.014** | 0.018± 0.012** | 0.523± 0.244** |
| X02 low | 488 | 0.101± 0.076 | 0.061± 0.041 | 0.078± 0.049 | 0.084± 0.063 | 0.099± 0.060 | 0.099± 0.085 | 0.076± 0.091 | 0.051± 0.051 | 0.037± 0.034* | 0.035± 0.025* | 0.608± 0.245* |
| Positive control | 360 | 0.074± 0.086 | 0.065± 0.052 | 0.059± 0.065 | 0.073± 0.039 | 0.070± 0.075 | 0.089± 0.077 | 0.053± 0.057 | 0.042± 0.043 | 0.044± 0.052 | 0.021± 0.043 | 0.588± 0.356* |
| Model | - | 0.169± 0.122 | 0.044± 0.047 | 0.083± 0.065 | 0.094± 0.066 | 0.126± 0.078 | 0.085± 0.070 | 0.068± 0.066 | 0.084± 0.071 | 0.073± 0.051 | 0.070± 0.044 | 0.896± 0.413 |
| Blank | - | 0.011± 0.007** | 0.010± 0.006* | 0.011± 0.008** | 0.012± 0.008** | 0.016± 0.007** | 0.018± 0.007** | 0.013± 0.008** | 0.018± 0.011** | 0.016± 0.009** | 0.016± 0.009** | 0.141± 0.038** |

| | | | | | | | | | | | | |
|---|---|---|---|---|---|---|---|---|---|---|---|---|
| Note: in comparison with the model group, *: P < 0.05; **: P < 0.01 | | | | | | | | | | | | |

### Test Example 5 Effects of the drugs on rat myocardial ischemia caused by pituitrin (PIT).

### Materials and methods

Animal: SD rats in clean grade, 5-6 week-old, body weight of 200-250 g, both males and females used.

Medicament: Y01 (echinocystic acid:oleanolic acid = 3:1, prepared by the method in Example 4, in which the total content of the echinocystic acid and the oleanolic acid is 97.4%), in-house-made off-white powder; echinocystic acid EA (containing 98.4% of echinocystic acid), in-house-made pure-white powder; oleanolic acid OA (containing 97.8% of oleanolic acid), in-house-made pure-white powder; pituitrin, Lot number: 080315, Bengbu Hongye Biochemical pharmaceuticals Factory product; sodium pentobarbital, manufactured by Chengdu Changzheng Huabo Co., Ltd.; Tongxinluo capsule, the product from Shijiazhuang Yiling Pharmaceutical Co., Ltd.

Instruments: ASB240U Biosignal Acquisiton System, Chengdu Aosheng Electronics Co., Ltd; KQ5200B Ultrasonic Cleaner, Kunshan Ultrasonic Instrument Co. Ltd.; Milli-Q Gradient A10 Pure Water Device: USA Millipore Co.; AB 104-N Electronic Balance: METTLER TOLEDO Co..

Procedure: 90 SD rats sensitive to pituitrin (PIT) and having normal ECG were screened, who were 5 - 6 weeks old and have body weight of 200-240 g. They were assigned randomly into six groups: the model group, the blank control group, the positive control group (Tongxinluo capsule of 360 mg/kg), Y01 group (68.4 mg/kg, in which the content of echinocystic acid was 50 mg/kg and the content of oleanolic acid was 16.7 mg/kg), EA group (50.8 mg/kg, the content of echinocystic acid was 50 mg/kg), OA group (17.0 mg/kg, the content of oleanolic acid was 16.7 mg/kg)), with 15 animals per group evenly composed of males and females. Each animal in all groups was intragastrically administered with the corresponding drug at a volume of 10 ml/kg, for 7 successive days. The rat was injected intraperitoneally with 1% sodium pentobarbital (30 mg/kg) 100 min after the final administration. When anesthetized, the animal was immobilized onto a frog board and subjected to trace of normal electrocardiogram over 2 min with Lead II. Then, PIT (1.5 IU/kg) was injected slowly over 20 s into the rat through its tail vein. The changes in the electrocardiogram were traced in 15 min after PIT injection. The data from the ST segment were collected at the time-points of 10 s, 30 s, 1 min, 2 min, 4 min, 6 min, 8 min, 10 min, 13 min, and 15 min. ST segment shifts at the various time-points after modeling were compared. The data in the experiment were processed with the statistical software package SPSS, Version 13.0. The measured data were indicated as mean ± standard deviation ( X ±*S.D.).* The comparisons among multiple groups were tested by ANOVA for randomized block design, and the comparison between two groups were tested by SNK-q test.

### Results:

It is known from analyzing the sum of ST segment shift in Table 8 that both Y01 and EA can antagonize ST segment shift, in which the sum of ST segment shift for Y01 is the most significant (P <0.01) as compared to that of the model group, while the antagonism against ST segment shift for OA is not apparent, as compared its sum of ST segment shift with that of the model group (P>0.05), and with that of the Y01 group (P<0.05). According to the values of ST segment shift in the myocardial ischemia model at various time-points, both Y01 and EA exhibit a significant effect of anti-myocardial ischemia at almost all time-points after injection of PIT, while OA exhibits an unapparent effect of anti-myocardial ischemia at each time-point after injection of PIT.

**Table 8 Effects on ST segment of the electrocardiogram in rat with an acute myocardial ischemia caused by PIT ( x ± s, n = 15).**

| Group | Dosage (mg/kg) | ΔST(mV) | | | | | | | | | | |
|---|---|---|---|---|---|---|---|---|---|---|---|---|
| | | 10 sec | 30 sec | 1 min | 2 min | 4 min | 6 min | 8 min | 10 min | 13 min | 15 min | Σ |
| Y01 | 68.4 | 0.057± 0.039** | 0.049± 0.030 | 0.047± 0.034* | 0.051± 0.044* | 0.049± 0.045* | 0.053± 0.051* | 0.049± 0.036* | 0.035± 0.031* | 0.031± 0.036* | 0.036± 0.029* | 0.447± 0.272** |
| EA | 50.8 | 0.089± 0.089* | 0.060± 0.053 | 0.064± 0.032* | 0.075± 0.063 | 0.060± 0.039* | 0.063± 0.045* | 0.070± 0.048 | 0.061± 0.042* | 0.055± 0.039* | 0.056± 0.048 | 0.653± 0.367* |
| OA | 17.0 | 0.132± 0.113 | 0.068± 0.044 | 0.079± 0.052 | 0.061± 0.034* | 0.082± 0.055 | 0.080± 0.067 | 0.073± 0.037 | 0.051± 0.032* | 0.060± 0.037 | 0.053± 0.042 | 0.739± 0.408Δ |
| Positive control | 360 | 0.070± 0.064* | 0.061± 0.043 | 0.062± 0.042* | 0.060± 0.031* | 0.056± 0.037* | 0.051± 0.046* | 0.053± 0.035* | 0.056± 0.064 | 0.045± 0.038* | 0.035± 0.033* | 0.549± 0.323* |
| Model | - | 0.155± 0.102 | 0.064± 0.057 | 0.088± 0.056 | 0.090± 0.053 | 0.092± 0.058 | 0.091± 0.063 | 0.085± 0.042 | 0.092± 0.058 | 0.082± 0.061 | 0.077± 0.036 | 0.916± 0.485 |
| Blank | - | 0.010± 0.006** | 0.008± 0.005* | 0.012± 0.007** | 0.012t 0.008** | 0.010± 0.006** | 0.010± 0.008** | 0.015± 0.009** | 0.013± 0.012** | 0.010± 0.004** | 0.009± 0.006** | 0.109± 0.036** |

| | | | | | | | | | | | | |
|---|---|---|---|---|---|---|---|---|---|---|---|---|
| Note: in comparison with the model group, *: P < 0.05; **: P < 0.01; in comparison with Y01group, Δ: P<0.05. | | | | | | | | | | | | |

Data in Table 8 indicate that both the gleditsiae extract Y01 and the echinocystic acid sample have a significant effect of anti-myocardial ischemia, and can be used in early and recovery periods of coronary heart attack, while oleanolic acid have an improvement on myocardial ischemia but not significant. Considering the intensity of the actions, the gleditsiae extract Y01 is apparently superior over either echinocystic acid or oleanolic acid.

### Test Example 6 Effects of the pharmaceutical composition of the invention on rat arrhythmia induced by barium chloride

### Materials and methods

Animal: SD rats in clean grade, 5-6 week-old, body weight of 200-250 g, both males and females used.

Medicament: X01 (echinocystic acid:oleanolic acid = 3:1, in which the total content of the echinocystic acid and the oleanolic acid is 92.8%), in-house-made off-white tablet, Lot number: 090309; X02 (echinocystic acid:oleanolic acid = 1:1, in which the total content of the echinocystic acid and the oleanolic acid is 98.8%), in-house-made off-white tablet, Lot number: 090310; barium chloride, A.P, from Kaiyuan Chemical Reagent Factory, Lot number: 0807002; CTRSR, manufactured by Sichuan Shuzhong Pharmaceutical Co., Ltd., brown tablet, Lot number: 080202.

Instruments: ASB240U Biosignal Acquisiton System, Chengdu Aosheng Electronics Co., Ltd; KQ5200B Ultrasonic Cleaner, Kunshan Ultrasonic Instrument Co. Ltd.; Milli-Q Gradient A10 Pure Water Device, USA Millipore Co.; AB 104-N Electronic Balance, METTLER TOLEDO Co..

Procedure: 112 SD rats sensitive to barium chloride and having normal ECG were screened, who were 5 - 6 weeks old and had body weight of 200-250 g. They were assigned randomly into six groups: the model group, the positive control group (Compound Tablet of Red Sage Root of 75 mg/kg), X01 high dose group (69.5 mg/kg), X01 low dose group (34.7 mg/kg), X02 high dose group (97.7 mg/kg), X02 low dose group (48.8 mg/kg), with 15 animals per group evenly composed of males and females. For purpose of prevention, each animal in all groups was subjected to intragastrical administration with the corresponding drug, q.d. for 5 successive days. The rat was anesthetized by injection of 10% chloral hydrate (400 mg/kg) after the final administration, immobilized in the supine position and left still for 30 min, followed by tracing a period of normal electrocardiogram in Lead II. Arrhythmia in the rat was induced by injection of 0.4% barium chloride at 4 mg/kg through lingual vein in 45 min after the final administration. The changes in Lead II electrocardiogram were observed. Time points for occurrence of bidirectional ventricular arrhythmia (early or late) and durations thereof were considered as indicators. Duration of bidirectional ventricular arrhythmia lasting for more than 30 min was recorded as 30 min.

### Results:

It can be seen from Table 9 that the tested drugs were able to put off the time point for occurrence of bidirectional ventricular arrhythmia in rat resulting from intravenous injection of barium chloride and to reduce duration of this arrhythmia. Moreover, efficacies of the prophylactic treatments were presented in a gradient manner, showing clear dose-effect relationship. Though the efficacies in the dose groups have not significantly difference as compared with that in the positive control (CTRSR) group, it can be seen from the data in Table 9 that the high dose groups of the pharmaceutical compositions X01 and X02 are slightly better than the CTRSR group in afterponing occurrence of arrhythmia and reducing duration thereof. This suggests that the pharmaceutical composition of the invention have advantages in treating arrhythmia.

**Table 9 Prophylactic effect of the pharmaceutical composition of the invention against rat arrhythmia induced by BaCl₂ ( x̅ ± s, n = 15)**

| Group | Dosage (mg/kg) | Bidirectional arrhythmia | |
|---|---|---|---|
| | | Time of occurrence (sec) | Duration (sec) |
| X01 high | 69.5 | 78.8±31.5** | 282.1±447.2** |
| X01 low | 34.7 | 60.6±25.3* | 502.2±332.4** |
| X02 high | 97.7 | 80.9±29.8** | 312.8±396.S** |
| X02 low | 48.8 | 64.7±22.6* | 481±388.4** |
| Positive control | 42 | 77.4±24.3** | 334.2±470.2** |
| Model | - | 39.1±15.4 | 1282±545.7 |

| | | | |
|---|---|---|---|
| Note: in comparison with the model group, *: P < 0.05; **: P < 0.01 | | | |

### Test Example 7 Effect of the pharmaceutical composition of the invention and echinocystic acid on lipid disorders in rabbit

### Materials and methods

Animal: purebred Japanese white rabbit, Grade I, male, 3 month-old, 1.5 - 2.0 kg per animal.

Medicament: Z01 (echinocystic acid : oleanolic acid = 3:1, in which the total content of the echinocystic acid and the oleanolic acid is 99.0%), in-house-made off-white tablet, Lot number: 090205; Z02 (echinocystic acid : oleanolic acid = 1:1, in which the total content of the echinocystic acid and the oleanolic acid is 94.2%), in-house-made off-white tablet, Lot number: 090206; atorvastatin calcium tablet (Lipitor), manufactured by Pfizer Inc., Lot number: 95837012; fenofibrate capsule (Lipanthyl), manufactured by Laboratories Fournier S.A. France, Lot number: 12094; physiological saline, provided by Chengdu Qingshan Likang Pharmarceutical Co., Ltd., clear colorless liquid, Lot number: A0703025.

Instruments: fully automated biochemistry analyzer (Roche Integra 400 Plus, Switzerland); TGL-16G centrifuge; AB104-N electronic balance; BP211D electronic balance.

Procedure: Animals were subjected to acclimation feeding for 10 days. Then, 60 animals were raised with high-fat feed (consisting of 1 % cholesterol + 5% refined lard + 10% egg yolk powder + 84% basal feed) and 6 additional animals were raised with the basal feed. After raised for 15 days, the animals were subjected to blood sampling from central ear artery while fasting. The sera were separated and analyzed for various blood lipid indices. From the sixty animals raised with the high-fat feed, 42 animals were selected on the basis of TC content and assigned into seven groups by randomized block design with six animals per group; the six animals raised with the basal feed were used as the blank control group. After grouping, animals in all of the groups were all raised with the basal feed and began to be intragastrically administered according to the group schedule of: low dose Z01 group (8.4 mg/kg), high dose Z01 group (16.8 mg/kg), low dose Z02 group (13.3 mg/kg), high dose Z02 group (26.5 mg/kg), Lipitor group (2.5 mg/kg), Lipanthyl group (20 mg/kg), the model group, and the blank control group. Administration lasted for 4 weeks. The data in the experiment were treated with the statistical software package SPSS, Version 13.0. The measured data were expressed as mean ± standard deviation( X ± *S.D.).* The comparisons among multiple groups were tested by ANOVA for randomized block design, and the comparisons between two groups were tested by SNK-q test.

### Results:

Blood samples were collected from the fasting animals in all of the groups of the experiment prior to administration and in 24 h after the final administration and assayed for content of total cholesterol (TC) and triglyceride (TG). The data were statistically treated and shown in Table 10.

**Table 10 Effect of the drug in the individual groups on serum TC and TG levels ( x ± s, n =6).**

| Group | Dosage (mg/kg) | Serum TC (mmol/1) | | Serum TG (mmol/l) | |
|---|---|---|---|---|---|
| | | prior to administration | After administration | prior to administration | After administration |
| Z01 high | 16.8 | 13,79±4.08 | 4.02±2.12** | 2.22±1.04 | 1,20±0.69** |
| Z01 low | 8.4 | 13.64±4.67 | 6.32±3.20* | 1.78±0.68 | 1.13±0.54** |
| | | | | | |
| Z02 high | 26.5 | 13.88±4.76 | 5.84±3.78* | 1.94±1.32 | 1.32±0.58* |
| Z02 low | 13.3 | 14.07±3.92 | 8.83±4.310△▲ | 2.07±0.44 | 1.66±1.24 |
| Lipitor | 2.5 | 14.135.34 | 3.86±2.07** | 1.58±0.54 | 1.12±0.44* |
| Lipanthyl | 20 | 13.76±4.78 | 3.45±1.63** | 2.18±1.22 | 1.08±0.53** |
| Model | - | 13,94±4.90 | 12.33±4.09 | 2.09±1.14 | 2.14±1.06 |
| Blank | - | 1.94±0.87 | 2.06±0.74 | 0.75±0.34 | 0.88±0.37 |

| | | | | | |
|---|---|---|---|---|---|
| Note: *Difference of the mean is of statistical significance as compared to that of the model group (P <0.05); **Difference of the mean is of statistical significance as compared to that of the model group (P <0.01); ^{^}Difference of the mean is of statistical significance as compared to that of the Lipitor group (P < 0.05); ^{▲}Difference of the mean is of statistical significance as compared to that of the Lipanthyl group (P < 0.05); | | | | | |

In Table 10, the result of the effects of the tested drugs on serum TC level in the groups shows that the pharmaceutical compositions in the high dose Z01 group, the low dose Z01 group, and the high dose Z02 group have efficacies in adjusting serum TC level of rabbit with lipid disorders in different extents, but have no significant difference in efficacy as compared to the Lipitor group and the Lipanthyl group (P >0.05). Considering the trends on potency of reducing serum TC levels, the pharmaceutical compositions have different potencies as the high dose Z01 group > the high dose Z02 group > the low dose Z01 group > the Z02 low dose group. Collectively, the first three samples have the efficacy in adjusting and improving lipid disorders in rabbit.

The result of the effects of the tested drugs on serum TG levels in the groups shows that the pharmaceutical compositions in the high dose Z01 group, the low dose Z01 group, and the high dose Z02 group have efficacies in adjusting serum TG levels of rabbit with lipid disorders in different extents, but have no significant difference in efficacy as compared to the Lipitor group and the Lipanthyl group (P >0.05). Considering the trends on potency of reducing serum TG levels, the pharmaceutical compositions have different potencies as the high dose Z01 group > the low dose Z01 group > the high dose Z02 group.

### Test Example 8 Comparison of Effects on lipid disorders in the rabbit

### Materials and methods

Animal: purebred Japanese white rabbit, Grade I, male, 3 month-old, 1.5 - 2.0 kg per animal.

Medicament: Y01 (echinocystic acid:oleanolic acid = 3:1, the total content of the echinocystic acid and the oleanolic acid is 97.4%), in-house-made off-white powder; echinocystic acid EA (containing 98.4% of echinocystic acid), in-house-made pure-white powder; oleanolic acid OA (containing 97.8% of oleanolic acid), in-house-made pure-white powder; atorvastatin calcium tablets (Lipitor), manufactured by Pfizer Inc.; physiological saline, manufactured by Sichuan Meida Kangjiale Pharmaceutical Co, Ltd..

Instruments: a fully automated biochemistry analyzer (Roche Integra 400 Plus, Switzerland); TGL-16G centrifuge; AB104-N electronic balance; BP211 D electronic balance.

Procedure: Animals were subjected to acclimation feeding for 10 days. Then, 40 of the animals were raised with a high-fat feed (consisting of 1% cholesterol +5 % refined lard +10 % egg yolk powder + 84% basal feed) and 6 of additional animals were raised with basal feed. After raised with the feed for 15 days, the animals were subjected to blood sampling from central ear artery while fasting. The sera were separated and analyzed for various blood lipid indices. From 40 animals raised with the high-fat feed, 30 animals were selected on the basis of TC content and assigned into five groups by randomized block design with six animals per group; six animals raised with the basal feed were used as the blank control group. After grouping, animals in the individual groups were all raised with the basal feed and began to be intragastrically administered according to the group schedule of: the Y01 group (16.4 mg/kg, in which the amount of echinocystic acid was 2 mg/kg and the amount of oleanolic acid was 4 mg/kg), the EA group (12.2 mg/kg, in which the amount of echinocystic acid was 12 mg/kg), the OA group (4.0 mg/kg, in which the amount of oleanolic acid was 4 mg/kg), the Lipitor group (2.5 mg/kg), the model group, and the blank control group. Administration lasted for 4 weeks. The data in the experiment were treated with the statistical software package SPSS, Version 13.0. The measured data were indicated as mean ± standard deviation ( *X̅* ± *S*.*D*.). ANOVA of randomized block design was used for comparisons among multiple groups, and SNK-q test was used for comparisons between two groups.

### Results:

Blood samples were collected from the fasting animals in each of the groups of the experiment prior to the administration and in 24 h after the final administration and assayed for the content of total cholesterol (TC) and triglyceride (TG). The data were statistically treated and shown in Table 11.

**Table 11 Effect of the drugs in each of the groups on serum TC and TG levels ( x̅ ± s, n =6).**

| Group | Dosage (mg/kg) | Serum TC (mmol/l) | | Serum TG (mmol/l) | |
|---|---|---|---|---|---|
| | | prior to administration | After administration | prior to administration | After administration |
| Y01 | 16.4 | 14.98±4.98 | 5.30±2.76** | 2.32±1.28 | 1.10±0.64** |
| EA | 12.2 | 15.07±5.48 | 9.77±6.30* | 2.17±1.04 | 1.64±0.89* |
| OA | 4.0 | 14.97±5.22 | 12.05±6.48△ | 2.06±0.44 | 1.83±1.02 |
| Positive control | 2.5 | 15.06±6.26 | 5.06±1.89** | 2.44±1.37 | 1.54±0.79* |
| Model | - | 14.94±5.79 | 13.24±5.66 | 2.38±0.85 | 1.92±1.42 |
| Blank | - | 1.88±0.74 | 1.64±0.57 | 0.90±0.45 | 0.86±0.50 |

| | | | | | |
|---|---|---|---|---|---|
| Note: in comparison with the model group, *: P < 0.05; **: P < 0.01; in comparison with the Y01 group, △: P<0.05. | | | | | |

In Table 11, the results of the effects of the tested drugs on serum TC level in each of the groups show that the Y01 group and the EA group have different efficacies in adjusting the serum TC level of rabbit with lipid disorders, as compared to the model group they show significant differences (P < 0.01 or P < 0.05) after administration; the OA group has unapparent efficacy in adjusting the serum TC level of rabbit with lipid disorders, as compared to the model group after administration, it shows no significant difference (P>0.05) and compared to the Y01 group, it shows significant difference of P< 0.05.

The results of the effects of the tested drugs on serum TG level in each of the groups show that the Y01 group and the EA group have different efficacies in adjusting the serum TG level of rabbit with lipid disorders, as compared to the model group they show significant differences (P < 0.01 or P < 0.05); the OA group has unapparent efficacy in adjusting the serum TG level of rabbit with lipid disorders, as compared to the model group, it shows no significant difference (P>0.05) and compared to the Y01 group, it shows significant difference of P < 0.05.

In view of the above, the drug used for the Y01 group and echinocystic acid have apparent efficacies in adjusting and improving lipid disorders in rabbit. However, oleanolic acid has no apparent efficacy in improving lipid disorders in rabbit. Considering the intensities of the actions, the drug used for the Y01 group is apparently superior over echinocystic acid and oleanolic acid.

### Test Example 9 Prophylactic effect of the pharmaceutical composition of the invention against rabbit atherosclerosis

### Materials and methods

Animal: purebred Japanese white rabbit, Grade I, male, 3 month-old, 1.5 - 2.0 kg per animal.

Medicament: Z01 (echinocystic acid:oleanolic acid = 3:1, in which the total content of the echinocystic acid and the oleanolic acid is 99.0%), in-house-made off-white tablet, Lot number: 090205; Z02 (echinocystic acid:oleanolic acid = 1:1, in which the total content of the echinocystic acid and the oleanolic acid is 94.2%), in-house-made off-white tablet, Lot number: 090206; atorvastatin calcium tablet (Lipitor), manufactured by Pfizer Inc., Lot number: 95837012; fenofibrate capsule (Lipanthyl), manufactured by Laboratoires Fournier S.A. France, Lot number: 12094; physiological saline, provided by Chengdu Qingshan Likang Pharmarceutical Co., Ltd., clear colorless liquid, Lot number: A0703025; bovine serum albumin (BSA), manufactured by Sigma Corporation, USA.

Instruments: a fully automated biochemistry analyzer (Roche Integra 400 Plus, Switzerland); TGL-16G centrifuge; AB104-N electronic balance; BP211D electronic balance; the electric glass homogenizer DY89-II.

Procedure: After acclimation feeding for 10 days, 50 of animals were completely randomly assigned into groups of: the Z01 group (16.8 mg/kg), the Z02 group (26.5 mg/kg), the Lipitor group (2.5 mg/kg), the Lipanthyl group (20 mg/kg), and the model group, with 9 animals per group; the blank control group consisting of 6 animals. After grouping, the animals in the dose groups and the model group were raised daily with a high-fat feed (consisting of 1 % cholesterol + 5% refined lard + 10% egg yolk powder + 84% basal feed), while the animals in the blank control group were raised with basal feed. At same time, all animals were subjected to intragastric administration according the group schedule for 12 weeks. At the end of the week 4 during administration, the animals were subjected to injection of 10% BSA once through rabbit marginal ear vein at a dose of 200 mg /kg to facilitate establishment of the animal AS model. The data in the experiment were treated with the statistical software package SPSS, Version 13.0. The measured data were indicated as mean ±standard deviation( *X̅* ± *S.D.*). The comparisons among multiple groups were tested by one-way analysis of variance for complete randomized block design, and the comparisons between two groups were tested by SNK-q test. Statistics of nonparametric information were performed with Kruskal-wallis H test and the comparisons thereof between two groups were tested by Nemenyi test.

### Results:

### 1. Effects on the lipid level in tissue homogenate

(1) Each animal in the groups of the experiment was sacrificed in 24 after the final administration. The aorta was removed and homogenized for determination of lipid levels therein. The data were statistically treated and shown in Table 12.

**Table 12. Effects on the lipid level in aortic tissue homogenate in each of the dose groups ( X̅ ± S.D., n=6).**

| Group | Dosage (mg/kg) | Lipid level in aortic tissue homogenate (mmol/100g tissue) | |
|---|---|---|---|
| | | TC | TG |
| Z01 group | 16.8 mg/kg | 1.38±0.93* | 1.04±0.42* |
| Z02 group | 26.5 mg/kg | 1.76±0.36* | 1.36±0.39* |
| Lipitor group | 2.5 mg/kg | 1.47±0.36* | 0.94±0.53* |
| Lipanthyl group | 20 mg/kg | 1.34±0.64* | 0.91±0.33* |
| Model group | - | 4.99±2.62 | 2.27±0.91 |
| Blank group | - | 0.35±0.06 | 0.70±0.11 |

| | | | |
|---|---|---|---|
| Note: *Difference of the mean is of statistical significance as compared to that of the model group (P <.0.05). | | | |

In Table 12, the results of effects of the tested drugs in each group on TC levels in aortic tissue homogenate shows that the Z01 group and the Z02 group have efficacies to different extent in reducing the TC levels in aorta of rabbit with lipid disorder, which are of no significant difference as compared to those of the Lipitor and the Lipanthyl groups (P>0.05). Considering the trend on potency of reducing TC level in aorta, the potency of the pharmaceutical composition of the Z01 group > the potency of the pharmaceutical composition of the Z02 group.

The results of effects of the tested drugs in each group on TG levels in aortic tissue homogenate shows that the Z01 group and the Z02 group have efficacies to different extent in reducing the TG levels in aorta of rabbit with lipid disorder, which are of no significant difference as compared to those of the Lipitor and the Lipanthyl groups (P>0.05). Considering the trend on potency, the potency of the pharmaceutical composition of the Z01 group > the potency of the pharmaceutical composition of the Z02 group.

It can be seen from the effects of the tested drugs in each experiment group on the lipid level in aortic tissue homogenate, the pharmaceutical compositions of the invention, both Z01 and Z02, have the effects in reducing the lipid level in aorta, which is embodied as improving scleratheroma due to high-fat diet.
(2) Each animal in each group of the experiment was sacrificed in 24 h after the final administration. The liver was removed and homogenized for determination of lipid levels therein. The data were statistically treated and shown in Table 13.

**Table 13. Effects on the lipid level in hepatic tissue homogenate in each of the dose groups ( X̅ ± S.D., n=6).**

| Group | Dosage (mg/kg) | Lipid level in hepatic tissue homogenate (mmol/100g tissue) | |
|---|---|---|---|
| | | TC | TG |
| Z01 group | 16.8 mg/kg | 1.39±0.70* | 0.61±0.42* |
| Z02 group | 26.5 mg/kg | 1.59±1.08* | 0.80±0.58* |
| Lipitor group | 2.5 mg/kg | 1.24±0.53* | 0.51±0.38* |
| Lipanthyl group | 20 mg/kg | 2.46±0.13* | 1.27±0.14* |
| Model group Blank | - | 5.60±2.94 | 3.95±2.61 |
| group | - | 0.33±0.08 | 0.05±0.02 |

| | | | |
|---|---|---|---|
| Note: *Difference of the mean is of statistical significance as compared to that of the model group (P < 0.05). | | | |

In Table 13, the results of effects of the tested drugs in each group on TC levels in hepatic tissue homogenate shows that the Z01 group and the Z02 group have efficacies to a different extent in reducing the TC level in hepatic tissue of rabbit with lipid disorder, which are of no significant difference as compared to the Lipitor and the Lipanthyl groups (P >0.05). Considering the trend on potency of reducing TC levels in hepatic tissue, the potency of the pharmaceutical composition of Z01 group > the potency of the pharmaceutical composition of Z02 group.

The results of effects of the tested drugs in each group on LDL-C levels in hepatic tissue homogenate shows that the Z01 group and the Z02 group have efficacies to a different extent in reducing the LDL-C level in hepatic tissue of rabbit with lipid disorder, which are of no significant difference as compared to the Lipitor and the Lipanthyl groups (P >0.05). Considering the trend on potency, the potency of the pharmaceutical composition of Z01 group > the potency of the pharmaceutical composition of Z02 group.

It can be seen from the effects of the tested drugs in each experiment group on the lipid level in hepatic tissue homogenate, the pharmaceutical compositions of the invention have an effect in reducing the cholesterol level in hepatic tissue, which is embodied as inhibiting synthesis of cholesterol in hepatic tissue.

### 2. Effects on area of atherosclerotic plaque in aorta

Each animal in each group of the experiment was sacrificed in 24 h after the final administration. The aorta was removed and stained grossly. Percentages of the area of the atherosclerotic plaque was calculated and statistically treated. The data were shown in Table 14.

**Table 14. Effects on area of atherosclerotic plaque in aorta in each of the dose groups ( X̅ ± S.D., n=6).**

| Group | Dosage (mg/kg) plaque | Percentages of area of the atherosclerotic |
|---|---|---|
| Z01 group | 16.8 mg/kg | 14.9%±5.0%* |
| Z02 group | 26.5 mg/kg | 21.0%±5.9%* |
| Lipitor group | 2.5 mg/kg | 14.1%±2.7%* |
| Lipanthyl group | 20 mg/kg | 15.2%±4.4%* |
| Model group | - | 51.8%±17.5% |
| Blank group | - | 0 |

| | | |
|---|---|---|
| Note: *Difference of the mean is of statistical significance as compared to that of the model group (P < 0.05). | | |

In Table 14, the results from the effects on area of the atherosclerotic plaque in the aorta in each of the experimental groups show that the Z01 group and the Z02 group have efficacies to a different extent in improving the atherosclerotic plaque in aorta, which are of no significant difference as compared to those of the Lipitor and the Lipanthyl groups (P>0.05). Considering the trend on the potency of improving the atherosclerotic plaque in aorta, the potency of the pharmaceutical composition Z01 group > the potency of the pharmaceutical composition Z02 group.

In a word, the pharmaceutical composition of the invention has an efficacy in preventing and improving atherosclerosis in rabbit.

### Test Example 10 Therapeutic effect of the pharmaceutical composition of the invention on rabbit atherosclerosis

### Materials and methods

Animal: purebred Japanese white rabbit, Grade I, male, 3 month-old, 1.5 - 2.0 kg per animal.

Medicament: Z01 (echinocystic acid:oleanolic acid = 3:1, in which the total content of the echinocystic acid and the oleanolic acid is 99.0%), in-house-made off-white tablet, Lot number: 090205; Z02 (echinocystic acid:oleanolic acid = 1:1, in which the total content of the echinocystic acid and the oleanolic acid is 94.2%), in-house-made off-white tablet, Lot number: 090206; atorvastatin calcium tablet (Lipitor), manufactured by Pfizer Inc., Lot number: 95837012; fenofibrate capsule (Lipanthyl), manufactured by Laboratoires Fournier S.A. France, Lot number: 12094; physiological saline, provided by Chengdu Qingshan Likang Pharmarceutical Co., Ltd., clear colorless liquid, Lot number: A0703025; Bovine serum albumin (BSA), manufactured by Sigma Corporation, USA.

Instruments: a fully automated biochemistry analyzer (Roche Integra 400 Plus, Switzerland); TGL-16G centrifuge; AB104-N electronic balance; BP211D electronic balance; OLYMPUS optical microscope; an inverted microscope (provided with a color imaging system), manufactured by Zeiss Corporation.

Procedure: After acclimation feeding for 10 days, 50 of animals were completely randomly assigned into groups of: the Z01 group (16.8 mg/kg), the Z02 group (26.5 mg/kg), the Lipitor group (2.5 mg/kg), the Lipanthyl group (20 mg/kg), and the model group, with 9 animals per group; the blank control group consisting of 6 animals. After grouping, animals in the dose groups and the model group were raised daily with a high-fat feed (consisting of 1 % cholesterol + 5% refined lard + 10% egg yolk powder + 84% basal feed), while the animals in the blank control group were raised with basal feed. At the end of the week 4 during the raise, the animals were subjected to injection of 10% BSA once through rabbit marginal ear vein at a dose of 200 mg /kg to facilitate establishment of the animal AS mode. Modeling was ended at the week 8, instead of the high-fat feed, the animals in each group were raised with the basal feed, and were subjected to intragastric administration according the group schedule for 12 weeks. The data in the experiment were treated with the statistical suit SPSS, Version 13.0. The measured data were indicated as mean ± standard deviation( X ± *S.D.).* The nonparametric data were performed with Kruskal-wallis H test and the comparisons thereof between two groups were tested by Nemenyi test.

### Results:

### 1. Effects on thickness of vascular intima-media

Each animal in each group of the experiment was sacrificed after the final administration. The thoracic aorta was stained grossly. Then, a small piece of the artery was cut transversely at the end proximal to heart, the positions 2 cm and 4 cm away from the proximal end to heart respectively, embedded in paraffin, sliced, and stained with HE. The ratios of thickness of tunica intima to that of tunica media were calculated. The data were statistically analyzed and shown in Table 15.

**Table 15. Effects on thickness of vascular intima-media in each of the dose groups ( X ± S.D., n=6).**

| Group | Dosage (mg/kg) | Thickness ratio of tunica intima to tunica media |
|---|---|---|
| Z01 group | 16.8mg/kg | 0.4±0.3* |
| Z02 group | 26.5mg/kg | 0.6±0.4* |
| Lipitor group | 2.5mg/kg | 0.4±0.2* |
| Lipanthyl group | 20mg/kg | 0.4±0.1* |
| Model group | - | 1.6±1.2 |
| Blank group | - | 0.03±0.01 |

| | | |
|---|---|---|
| Note: *Difference of the mean is of statistical significance as compared to that of the model group (P < 0.05); | | |

It can be concluded from the experimental results in Table 15 that both of the two experimental drugs in the sample groups have an efficacy in reducing the ratio of thickness of tunica intima to that of tunica media, which are of no significant difference as compared to those of the positive control groups (Lipitor and Lipanthyl groups). The efficacy is embodied as therapeutic effect in treatment of atherosclerosis.

### 2. Effect on histopathology of aorta

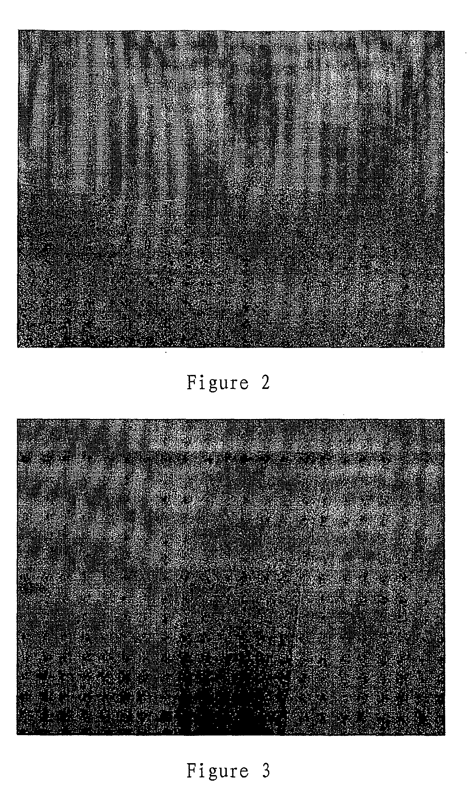

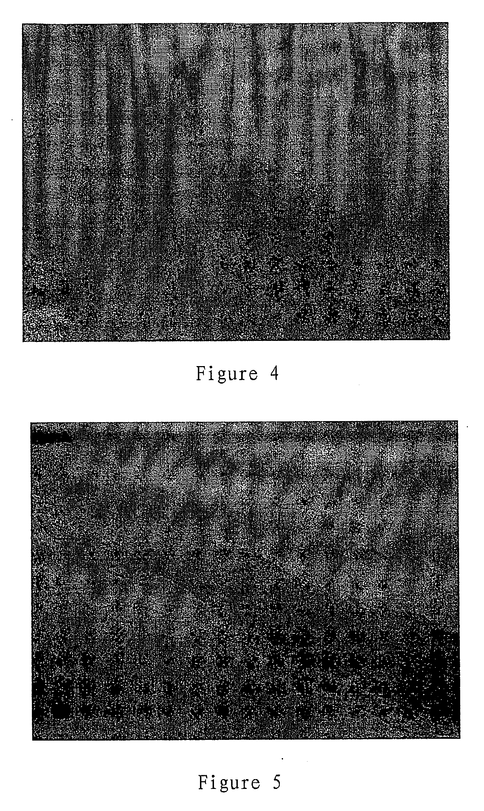

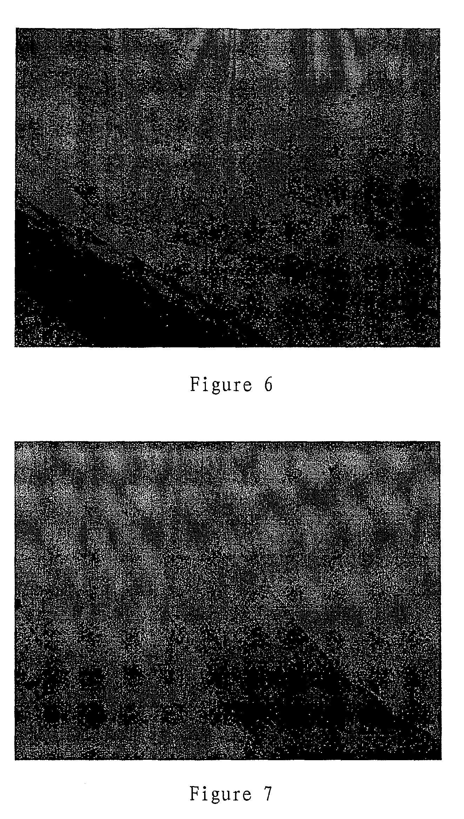

Each animal in each group of the experiment was sacrificed after the final administration. The thoracic aorta was stained grossly. Then, a small piece of the artery was cut transversely at the end proximal to heart, the positions 2 cm and 4 cm away from the proximal end to heart respectively, embedded in paraffin, sliced, stained with HE, and observed histopathologically. The cross sections of the aorta stained with HE were shown in Figs 2-7.

Significant endotheliosis of the aorta can be observed in the HE-stained specimens from in the model group, and the macrophage-derived foam cells carrying cholesterol lost their lipids during embedding and staining and thus appeared to be fluffy foam cells with the fluffy vacuoles left and distinct nucleus. The thicknesses of the Endotheliosis of aorta in the sample groups were thinner, showing improved anti-atherosclerosis effect, which were of no significant difference as compared to those in the Lipitor and Lipanthyl groups. Considering the trend of the potencies of the tested samples in improving the aortic endotheliosis, the potency of the pharmaceutical composition of the Z01 group > the potency of the pharmaceutical composition of the Z02 group.

In a word, the pharmaceutical composition of the invention has an efficacy in treating atherosclerosis in rabbit.

### INDUSTRIAL APPLICATION

The hepatotoxicity of the pharmaceutical composition is significantly decreased by controlling the ratio of echinocystic acid to oleanolic acid therein, which allows the pharmaceutical composition to be safer and more potential in treatment of cardiovascular disorders. By controlling the weight ratio of echinocystic acid to oleanolic acid in the gleditsiae extract, the optimized gleditsiae extract is obtained, which allows not only hepatotoxicity thereof to be reduced, but also the production cost thereof to be considerably reduced, thereby possessing active value, good prospect and suitability for industrial application.

## Claims

1. A pharmaceutical composition for treating cardiovascular disorders, **characterized in that**, the active ingredient thereof consists of:
3 to 1 part(s) by weight of echinocystic acid, and 1 part by weight of oleanolic acid.

2. The pharmaceutical composition for treating cardiovascular disorders according to Claim 1, **characterized in that**, the active ingredient thereof consists of:
3 to 2 parts by weight of echinocystic acid, and 1 part by weight of oleanolic acid.

3. The pharmaceutical composition for treating cardiovascular disorders according to Claim 1, **characterized in that**, the active ingredient thereof consists of:
3 parts by weight of echinocystic acid, and 1 part of oleanolic acid.

4. The pharmaceutical composition for treating cardiovascular disorders according to any one of Claims 1-3, **characterized in that**, said echinocystic acid and oleanolic acid are from a plant extract or chemical synthetic substances.

5. Use of the pharmaceutical composition according to any one of Claims 1-4 in preparation of a medicament for preventing or treating cardiovascular disorders.

6. The use according to Claims 5, **characterized in that**, the medicament is the one for preventing or treating coronary artery disease, angina pectoris, myocardial ischemia, myocardial infarction, arrhythmia, hyperlipidemia or atherosclerosis.

7. A gleditsiae extract, **characterized in that**, the gleditsiae extract contains echinocystic acid and oleanolic acid in a total content of 90.0%-99.9%, and echinocystic acid and oleanolic acid is presented in a weight ratio of 3:1-1:1.

8. The gleditsiae extract according to Claim 7, **characterized in that**, echinocystic acid and oleanolic acid is presented in a weight ratio of 3:1-2:1.

9. The gleditsiae extract according to Claim 8, **characterized in that**, echinocystic acid and oleanolic acid is presented in a weight ratio of 3:1.

10. A method for preparing the gleditsiae extract according to Claim 7, 8, or 9, comprising the steps of:
a. preparing a medicinal material of *Fructus Gleditsiae Sinensis,* and subjecting the medicinal material to decoction with water, then filtering, and concentrating the filtrate;
b. adding an acid together with water or aqueous ethanol into the concentrated filtrate allowing the concentration of the acid to be 2 mol/L and the content of the optional ethanol to be 1-45%, stirring and boiling the same for a hydrolysis, then filtering the hydrolyzed solution, washing the obtained filter cake with purified water until the pH of the effluent being above 6, then drying the washed filter cake at 80°C to yield crude gleditsia sapogenin;
c. dissolving the crude gleditsia sapogenin in 15-40 parts of 95% ethanol while being heated, adding 1-4% of pharmaceutically acceptable activated carbon therein, stirring and boiling the same for 30 min followed by filtering while hot; adjusting pH of the filtrate to 2-6, followed by recovering ethanol therefrom, then cooling and crystallizing, and drying the obtained crystal at 80°C to yield the gleditsiae extract.

11. Use of the gleditsiae extract according to any one of Claims 7-9 in preparation of a medicament for preventing or treating cardiovascular disorders.

12. The use according to Claims 11, **characterized in that**, the medicament is the one for preventing or treating coronary artery disease, angina pectoris, myocardial ischemia, myocardial infarction, arrhythmia, hyperlipidemia or atherosclerosis.
